# EUROPEAN PATENT APPLICATION

(11) **EP 4 455 299 A1**
(43) Date of publication of application: **30.10.2024**
(21) Application number: 21968668.0
(22) Date of filing: 24.12.2021
(51) Int. Cl.: C12Q 1/68, C40B 20/02, C40B 40/06, C12M 1/34, C12Q 1/6869, C12Q 1/6837

(54) **METHOD FOR GENERATING LABELED NUCLEIC ACID MOLECULAR POPULATION AND KIT THEREOF**

(71) Applicant: BGI Shenzhen, Shenzhen, Guangdong 518083 (CN)
(72) Inventor: LIAO, Sha, Shenzhen, Guangdong 518083 (CN); CHEN, Ao, Shenzhen, Guangdong 518083 (CN); ZHANG, Wenwei, Shenzhen, Guangdong 518083 (CN); XU, Xun, Shenzhen, Guangdong 518083 (CN); PENG, Jian, Shenzhen, Guangdong 518083 (CN); LI, Wenjiao, Shenzhen, Guangdong 518083 (CN); CHEN, Xi, Shenzhen, Guangdong 518083 (CN); FU, Defeng, Shenzhen, Guangdong 518083 (CN)
(74) Representative: Inspicos P/S
(86) International application number: PCT/CN2021/141195
(87) International publication number: WO 2023/115536

(57) **Abstract**

The present application relates to transcriptome sequencing and biomolecule space information detection. Specifically, the present application relates to a method for positioning and labeling a nucleic acid molecule, a method for constructing a nucleic acid molecule library for transcriptome sequencing, and a kit for implementing the method.

## Description

### Technical Field

The present application relates to the technical fields of transcriptome sequencing and biomolecule spatial information detection. Specifically, the present application relates to a method for positionally labeling nucleic acid molecules and a method for constructing a nucleic acid molecule library for transcriptome sequencing. In addition, the present application also relates to a nucleic acid molecule library constructed by the method, and a kit for implementing the method.

### Background Art

The spatial position of cells in tissues significantly affects their functions. To explore this spatial heterogeneity, it is necessary to quantify and analyze the genome or transcriptome of cells with knowledge of spatial coordinates. However, it is very laborious, expensive and has low precision to collect small tissue regions or even single cells for genome or transcriptome analysis. Therefore, it is necessary to develop a method that can achieve high-throughput detection of spatial information of biomolecules (e.g., localization, distribution, and/or expression of nucleic acids) at the single-cell level or even the sub-cellular level.

### Contents of the present application

The present application provides a new method for generating a labeled nucleic acid molecule population, as well as a method for constructing a nucleic acid molecule library based on this method and performing high-throughput sequencing.

Method for generating a population of labeled nucleic acid molecules

In one aspect, the present application provides a method for generating a labeled nucleic acid molecule population, which comprises the following steps:
(1) providing a biological sample and a nucleic acid array, wherein the nucleic acid array comprises a solid support, the solid support is coupled with multiple kinds of oligonucleotide probes, each kind of oligonucleotide probe comprises at least one copy, and the oligonucleotide probe comprises or consists of a consensus sequence X1, a tag sequence Y, and a consensus sequence X2 in the direction from 5' to 3', wherein,
   each kind of oligonucleotide probe has a different tag sequence Y, and the tag sequence Y has a nucleotide sequence unique to the position of the kind of oligonucleotide probe on the solid support;
(2) contacting the biological sample with the nucleic acid array so that the position of an RNA (e.g., mRNA) in the biological sample is mapped to the position of the oligonucleotide probe on the nucleic acid array; preprocessing the RNA (e.g., mRNA) in the biological sample to generate a first nucleic acid molecule population, wherein the preprocessing comprises:
   (i) using a primer A to perform reverse transcription of the RNA (e.g., mRNA) of the biological sample to generate an extension product as a first nucleic acid molecule to be labeled, thereby generating the first nucleic acid molecule population; wherein, the primer A comprises a consensus sequence A and a capture sequence A, the capture sequence A is capable of annealing to the RNA to be captured (e.g., mRNA) and initiating an extension reaction, and the consensus sequence A is located upstream of the capture sequence A (e.g., located at the 5'-end of the primer A);
      or,
   (ii) (a) using a primer A to perform reverse transcription of the RNA (e.g., mRNA) of the biological sample to generate a cDNA strand, the cDNA strand comprises a cDNA sequence, which is generated by reverse transcription primed by the primer A and is complementary to the RNA (e.g., mRNA), and a 3'-end overhang; wherein, the primer A comprises a consensus sequence A and a capture sequence A, and the capture sequence A is capable of annealing to the RNA (e.g., mRNA) to be captured and initiating an extension reaction; the consensus sequence A is located upstream of the capture sequence A (e.g., at the 5'-end of the primer A); and, (b) annealing a primer B to the cDNA strand generated in (a) and performing an extension reaction to generate a first extension product as a first nucleic acid molecule to be labeled, thereby generating the first nucleic acid molecule population; wherein, the primer B comprises a consensus sequence B, a complementary sequence of the 3'-end overhang, and optionally a tag sequence B; the complementary sequence of the 3'-end overhang is located at the 3'-end of the primer B; and the consensus sequence B is located upstream of the complementary sequence of the 3'-end overhang (e.g., located at the 5'-end of the primer B);
      or,
   (iii) (a) using a primer A' to perform reverse transcription of the RNA (e.g., mRNA) of the biological sample to generate a cDNA strand, the cDNA strand comprises a cDNA sequence, which is generated by reverse transcription primed by the primer A' and is complementary to the RNA (e.g., mRNA), and a 3'-end overhang; wherein, the primer A' comprises a capture sequence A, the capture sequence A is capable of annealing to the RNA (e.g., mRNA) to be captured and initiating an extension reaction; (b) annealing a primer B to the cDNA strand generated in (a), and performing an extension reaction to generate a first extension product; wherein, the primer B comprises a consensus sequence B, a complementary sequence of the 3'-end overhang, and optionally a tag sequence B; the complementary sequence of the 3'-end overhang is located at the 3'-end of the primer B; the consensus sequence B is located upstream of the complementary sequence of the 3'-end overhang (e.g., located at the 5'-end of the primer B); and, (c) providing an extension primer and using the first extension product as a template to perform an extension reaction to generate a second extension product as a first nucleic acid molecule to be labeled, thereby generating the first nucleic acid molecule population;
(3) contacting a bridging oligonucleotide with the product of step (2) under a condition allowing annealing, annealing (e.g., in-situ annealing) the bridging oligonucleotide to the oligonucleotide probe and the first nucleic acid molecule to be labeled which is at the corresponding position of the oligonucleotide probe, and ligating the first nucleic acid molecule and the oligonucleotide probe on the array annealed with the bridging oligonucleotide to obtain a ligation product as a second nucleic acid molecule with a positioning tag, thereby generating a second nucleic acid molecule population;

wherein, the bridging oligonucleotide comprises: a first region and a second region, and optionally a third region located between the first region and the second region, the first region is located upstream of the second region (e.g., located 5' of the second region); wherein,
the first region is capable of annealing to the whole or a part of the consensus sequence A of the primer A in step (2)(i) or step (2)(ii) or to the whole or a part of the consensus sequence B of the primer B in step (2)(iii);
the second region is capable of annealing to the whole or a part of the consensus sequence X2.

In certain embodiments, in step (3) of the method, when the first region and the second region of the bridging oligonucleotide are directly adjacent, the ligation of the first nucleic acid molecule to the oligonucleotide probe comprises: using a nucleic acid ligase to ligate the nucleic acid molecule hybridized with the first region and the nucleic acid molecule hybridized with the second region on the same bridging oligonucleotide, to obtain a ligation product as the second nucleic acid molecule with a positioning tag; or,
when the bridging oligonucleotide comprises the first region, the second region and the third region located between them, the ligation of the first nucleic acid molecule to the oligonucleotide probe comprises: using a nucleic acid polymerase to perform a polymerization reaction with the third region as a template, and using a nucleic acid ligase to ligate the nucleic acid molecule hybridized with the first region and the nucleic acid molecule hybridized with the third region and the second region on the same bridging oligonucleotide, to obtain a ligation product as the second nucleic acid molecule with a positioning tag. In certain embodiments, the nucleic acid polymerase does not have 5' to 3' exonucleolytic activity or strand displacement activity.

In certain embodiments, each kind of oligonucleotide probe comprises one copy.

In certain embodiments, each kind of oligonucleotide probe comprises a plurality of copies.

In certain embodiments, a region where each kind of oligonucleotide probe is coupled to the solid support is referred to as a microdot. It is easy to understand that when each kind of oligonucleotide probe comprises one copy, each microdot is coupled with one oligonucleotide probe, and the oligonucleotide probes of different microdots have different tag sequences Y; when each kind of oligonucleotide probe comprises a plurality of copies, each microdot is coupled with a plurality of oligonucleotide probes, the oligonucleotide probes in the same microdot have the same tag sequence Y, and the oligonucleotide probes in different microdots have different tag sequences Y.

In certain embodiments, the solid support comprises a plurality of microdots, each microdot is coupled with one kind of oligonucleotide probe, and each kind of oligonucleotide probe may comprise one or more copies.

In certain embodiments, the solid support comprises a plurality of (e.g., at least 10, at least 10², at least 10³, at least 10⁴, at least 10⁵, at least 10⁶, at least 10⁷, at least 10⁸, or more) microdots; in certain embodiments, the solid support comprises at least 10⁴ (e.g., at least 10⁴, at least 10⁵, at least 10⁶, at least 10⁷, at least 10⁸, at least 10⁹, at least 10¹⁰, at least 10¹¹, or at least 10¹²) microdots/mm^{2.}

In some embodiments, the spacing between adjacent microdots is less than 100 µm, less than 50 µm, less than 10 µm, less than 5 µm, less than 1 µm, less than 0.5 µm, less than 0.1 µm, less than 0.05 µm, or less than 0.01 µm.

In certain embodiments, the microdots have a size (e.g., equivalent diameter) of less than 100 µm, less than 50 µm, less than 10 µm, less than 5 µm, less than 1 µm, less than 0.5 µm, less than 0.1 µm, less than 0.05 µm, or less than 0.01 µm.

Embodiments comprising step (1), step (2)(i) and step (3)

In some embodiments, the method comprises step (1), step (2)(i) and step (3); wherein the ligation product obtained in step (3) is taken as the second nucleic acid molecule with a positioning tag, which comprises from 5' to 3': the consensus sequence X1, the tag sequence Y, the consensus sequence X2, optionally a complementary sequence of the third region of the bridging oligonucleotide, and the sequence of the first nucleic acid molecule to be labeled.

In certain embodiments, in step (2)(i) of the method, the capture sequence A is a random oligonucleotide sequence.

In some embodiments, in step (3), the ligation product derived from each copy of the same kind of oligonucleotide probe has a different capture sequence A, and the capture sequence A serves as a unique molecular identifier (UMI) of the second nucleic acid molecule.

In certain embodiments, the extension product (the first nucleic acid molecule to be labeled) in step (2)(i) comprises from 5' to 3': the consensus sequence A, and the cDNA sequence that is generated by reverse transcription primed by the primer A and is complementary to the RNA.

In certain embodiments, in step (2)(i) of the method, the capture sequence A is a poly(T) sequence or a specific sequence targeting a target nucleic acid.

In certain embodiments, the primer A further comprises a tag sequence A, such as a random oligonucleotide sequence, and the tag sequence A serves as a unique molecular identifier (UMI) of the second nucleic acid molecule.

In certain embodiments, the capture sequence A is located at the 3'-end of the primer A, and the consensus sequence A is located upstream of the tag sequence A (e.g., located at the 5'-end of the primer A).

In certain embodiments, in step (3), the ligation product derived from each copy of the same kind of oligonucleotide probe has a different tag sequence A as a UMI.

In some embodiments, the extension product described in step (2)(i) comprises from 5' to 3': the consensus sequence A, the tag sequence A, and the cDNA sequence that is generated by reverse transcription primed by the primer A and is complementary to the RNA.

Embodiments comprising step (1), step (2)(ii) and step (3)

In some embodiments, the method comprises step (1), step (2)(ii) and step (3); wherein the ligation product obtained in step (3) is taken as the second nucleic acid molecule with a positioning tag, which comprises from 5' to 3': the consensus sequence X1, the tag sequence Y, the consensus sequence X2, optionally a complementary sequence of the third region of the bridging oligonucleotide, and the sequence of the first nucleic acid molecule to be labeled.

In certain embodiments, in step (2)(ii)(a) of the method, the capture sequence A is a random oligonucleotide sequence.

In some embodiments, in step (3), the ligation product derived from each copy of the same kind of oligonucleotide probe has a different capture sequence A, and the capture sequence A serves as a unique molecular identifier (UMI) of the second nucleic acid molecule.

In certain embodiments, the first extension product (the first nucleic acid molecule to be labeled) in step (2)(ii) comprises from 5' to 3': the consensus sequence A, the cDNA sequence that is generated by reverse transcription primed by the primer A and is complementary to the RNA, the 3'-end overhang sequence, optionally a complementary sequence of the tag sequence B, and a complementary sequence of the consensus sequence B.

In certain embodiments, in step (2)(ii)(a), the capture sequence A is a poly(T) sequence or a specific sequence targeting a target nucleic acid.

In certain embodiments, the primer A further comprises a tag sequence A, such as a random oligonucleotide sequence, and the tag sequence A serves as a unique molecular identifier (UMI) of the second nucleic acid molecule.

In certain embodiments, the capture sequence A is located at the 3'-end of the primer A, and the consensus sequence A is located upstream of the tag sequence A (e.g., located at the 5'-end of the primer A).

In certain embodiments, in step (3), the ligation product derived from each copy of the same kind of oligonucleotide probe has a different tag sequence A as a UMI.

In certain embodiments, the first extension product (the first nucleic acid molecule to be labeled) in step (2)(ii) comprises from 5' to 3': the consensus sequence A, the tag sequence A, the cDNA sequence that is generated by reverse transcription primed by the primer A and is complementary to the RNA, the 3'-end overhang sequence, optionally a complementary sequence of the tag sequence B, and a complementary sequence of the consensus sequence B.

In certain embodiments, in the method, the primer A comprises a 5' phosphate at the 5'-end.

In certain embodiments, before step (3), the method further comprises processing (e.g., heat-processing) the product of step (2)(i) or step (2)(ii) to remove RNA.

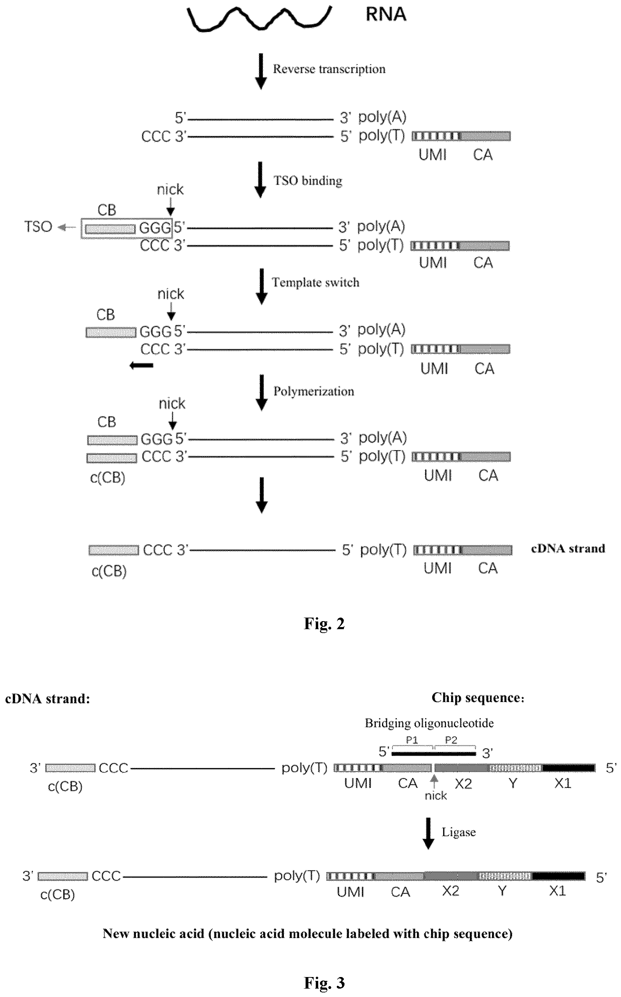

An exemplary embodiment of the present application comprising step (1), step (2)(ii) and step (3) is described in detail as follows:
1. An exemplary embodiment for preparing a cDNA strand using an RNA (e.g., mRNA) in a sample as a template comprises the following steps (as shown in Fig. 2):
   (1) A reverse transcriptase (e.g., a reverse transcriptase with terminal deoxynucleotidyl transferase activity) and a primer A are used to perform reverse transcription of the RNA molecule (e.g., mRNA molecule) in the permeabilized tissue sample to generate a cDNA, and an overhang (e.g., an overhang containing three cytosine nucleotides) is added to the 3'-end of the cDNA. Various reverse transcriptases with terminal deoxynucleotidyl transferase activity can be used to perform the reverse transcription reaction. In certain preferred embodiments, the reverse transcriptase used does not have RNase H activity.

The primer A comprises a poly(T) sequence and a consensus sequence A (marked as CA in the figure). In certain embodiments (e.g., when the method is used for constructing a 3' transcriptome library), the primer A further comprises a unique molecular identifier (UMI). Normally, the poly(T) sequence is located at the 3'-end of the primer A to initiate the reverse transcription. In a preferred embodiment, the UMI sequence is located upstream (e.g., 5') of the poly(T) sequence, and the consensus sequence A is located upstream (e.g., 5') of the UMI sequence.

(2) A template switching oligo (TSO, which can be used as a primer B, which comprises a consensus sequence B (marked as CB in the figure)) is used to anneal to or hybridize with the cDNA strand, subsequently, the nucleic acid fragment hybridized with or annealed to the TSO is extended by using the consensus sequence B as a template under the presence of a nucleic acid polymerase, so that a complementary sequence of the consensus sequence B is added to the 3'-end of the cDNA strand, thereby generating a nucleic acid molecule that carries at the 5'-end the consensus sequence A and the tag sequence A, and carries at the 3'-end a complementary sequence of the consensus sequence B.

The TSO sequence may comprise a sequence complementary to the 3'-end overhang of the cDNA strand. For example, when the cDNA strand comprises at the 3'-end an overhang of three cytosine nucleotides, the template switching oligo may comprise GGG at its 3'-end. In addition, the nucleotides of the TSO sequence can also be modified (e.g., the TSO sequence can be modified to comprise a locked nucleic acid) to enhance the binding affinity for the complementary pairing between the TSO sequence and the 3'-end overhang of the cDNA strand.

Without being limited by theory, various suitable nucleic acid polymerases (e.g., DNA polymerases or reverse transcriptases) can be used to perform the extension reaction, as long as they can extend the hybridized or annealed nucleic acid fragment (reverse transcription product) using the sequence of TSO or partial sequence thereof as a template. In certain exemplary embodiments, the hybridized or annealed nucleic acid fragment (reverse transcription product) can be extended using the same reverse transcriptase used in the aforementioned reverse transcription step.

In certain preferred embodiments, step (2) and step (1) are performed simultaneously.

In certain embodiments, the method optionally further comprises step (3): adding RNase H to digest the RNA strand in an RNA/cDNA hybrid to form a cDNA single strand.

In certain preferred embodiments, the method does not comprise step (3).

An exemplary structure of the cDNA strand prepared by the above exemplary embodiment comprises: the consensus sequence A, the UMI sequence, the sequence complementary to the sequence of the RNA (e.g., mRNA), and a complementary sequence of the consensus sequence B.

2. An exemplary embodiment, in which the 5'-end of the cDNA strand is labeled with an oligonucleotide probe (also referred as chip sequence) (that is, the 5'-end of the cDNA strand is ligated to the 3'-end of the chip sequence) to form a new nucleic acid molecule containing the chip sequence information (i.e., a nucleic acid labeled with chip sequence), comprises the following steps (shown in Fig. 3):
providing a bridging oligonucleotide, which comprises at 5'-end a sequence (a first region, P1) that is at least partially complementary to the 5'-end of the cDNA sequence (e.g., at least partially complementary to the consensus sequence A (CA)), and comprises at 3'-end a sequence (a second region, P2) that is at least partially complementary to the 3'-end of the chip sequence (e.g., at least partially complementary to the consensus sequence X2).

In certain preferred embodiments, the P1 and P2 sequences in the bridging oligonucleotide are directly adjacent, and there is no intermediate nucleotide between them.

In certain preferred embodiments, the P1 sequence, P2 sequence, consensus sequence A, and consensus sequence X2 each independently have a length of 20-100 nt (e.g., 20-70 nt). The bridging oligonucleotide is annealed or hybridized with the oligonucleotide probe and the cDNA strand, and then the 5'-end of the cDNA strand is ligated to the 3'-end of the oligonucleotide probe by a DNA ligase and/or a DNA polymerase, thereby generating a new nucleic acid molecule comprising the sequence information of the oligonucleotide probe (i.e., a nucleic acid molecule labeled with the oligonucleotide probe). In certain preferred embodiments, the DNA polymerase does not have 5' to 3' exonucleolytic activity or strand displacement activity.

An exemplary structure of a new nucleic acid molecule that comprises the chip sequence information as formed by the above exemplary embodiment comprises: the consensus sequence X1, the tag sequence Y, the consensus sequence X2, the consensus sequence A, the UMI sequence, a complementary sequence of the RNA (e.g., mRNA) sequence, and a complementary sequence of the consensus sequence B.

Embodiments comprising step (1), step (2) (iii) and step (3)

In some embodiments, the method comprises step (1), step (2) (iii) and step (3); wherein, the ligation product obtained in step (3) is taken as the second nucleic acid molecule with a positioning tag, which comprises from 5' to 3': the consensus sequence X1, the tag sequence Y, the consensus sequence X2, optionally a complementary sequence of the third region of the bridging oligonucleotide, and the sequence of the first nucleic acid molecule to be labeled.

In some embodiments, in step (2)(iii)(c) of the method, the extension primer is the primer B or a primer B', and the primer B' is capable of annealing to the whole or a part of a complementary sequence of the consensus sequence B and initiating an extension reaction.

In certain embodiments, in step (2)(iii)(c), the extension primer is the primer B'.

In certain embodiments, in step (2)(iii)(a) of the method, the capture sequence A of the primer A' is a random oligonucleotide sequence.

In certain embodiments, in step (2)(iii)(b), the primer B comprises the consensus sequence B, a complementary sequence of the 3' end overhang, and the tag sequence B.

In some embodiments, the first extension product comprises from 5' to 3': a cDNA sequence that is generated by reverse transcription primed by the primer A' and is complementary to the RNA sequence, the 3'-end overhang sequence, a complementary sequence of the tag sequence B, and a complementary sequence of the consensus sequence B; wherein the complementary sequence of the tag sequence B serves as a unique molecular identifier (UMI) of the second nucleic acid molecule.

In certain embodiments, in step (2)(iii)(c), the second extension product (the first nucleic acid molecule to be labeled) comprises from 5' to 3': the consensus sequence B or 3'-end partial sequence thereof, the tag sequence B, a complementary sequence of the 3'-end overhang sequence, and a complementary sequence of the cDNA sequence in the first extension product; wherein the tag sequence B serves as a unique molecular identifier (UMI) of the second nucleic acid molecule.

In certain embodiments, in step (2)(iii)(a) of the method, the capture sequence A of the primer A' is a poly(T) sequence or a specific sequence targeting a target nucleic acid.

In certain embodiments, the primer A' further comprises a tag sequence A, such as a random oligonucleotide sequence, and a consensus sequence A.

In certain embodiments, the capture sequence A is located at the 3'-end of the primer A'.

In certain embodiments, the consensus sequence A is located upstream of the capture sequence A (e.g., at the 5'-end of the primer A').

In certain embodiments, the primer B comprises the consensus sequence B, a complementary sequence of the 3'-end overhang, and the tag sequence B.

In certain embodiments, in step (2)(iii)(b), the first extension product comprises from 5' to 3': the consensus sequence A, optionally the tag sequence A, the cDNA sequence that is generated by reverse transcription primed by the primer A' and is complementary to the RNA, the 3'-end overhang sequence, a complementary sequence of the tag sequence B, and a complementary sequence of the consensus sequence B.

In certain embodiments, in step (2)(iii)(c), the second extension product (the first nucleic acid molecule to be labeled) comprises from 5' to 3': the consensus sequence B or 3'-end partial sequence thereof, the tag sequence B, a complementary sequence of the 3'-end overhang sequence, a complementary sequence of the cDNA sequence in the first extension product, optionally a complementary sequence of the tag sequence A, and a complementary sequence of the consensus sequence A.

In certain embodiments, in step (3), the ligation product derived from each copy of the same kind of oligonucleotide probe has a different tag sequence B as a UMI.

In certain embodiments, the extension primer comprises a 5' phosphate at the 5'-end.

In certain embodiments, before step (2)(iii)(c), the method further comprises: processing (e.g., heat-processing) the product of step (2)(iii)(a) or step (2)(iii)(b) to remove RNA.

In certain embodiments, in step (2)(iii)(b) of the method, the cDNA strand is annealed through its 3'-end overhang to the primer B, and, the cDNA strand is extended using the primer B as a template under the presence of a nucleic acid polymerase (e.g., a DNA polymerase or a reverse transcriptase) to generate the first extension product.

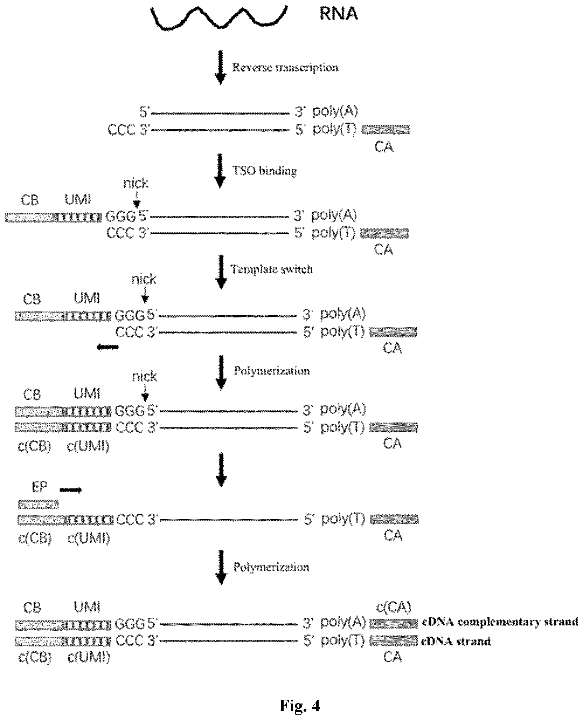

An exemplary embodiment of the present application comprising step (1), step (2) (iii) and step (3) is described in detail as follows:
1. An exemplary embodiment for preparing a complementary strand of a cDNA strand using an RNA (e.g., mRNA) in a sample as a template comprises the following steps (as shown in Fig. 4):
   (1) A reverse transcriptase (e.g., a reverse transcriptase with terminal deoxynucleotidyl transferase activity) and a primer A' are used for performing reverse transcription of the RNA molecule (e.g., mRNA molecule) in the permeabilized sample to generate a cDNA, and an overhang (e.g., an overhang containing three cytosine nucleotides) is added to the 3'-end of the cDNA. Various reverse transcriptases with terminal deoxynucleotidyl transferase activity can be used to perform the reverse transcription reaction. In certain preferred embodiments, the reverse transcriptase used does not have RNase H activity.
      The reverse transcription primer A' comprises a poly(T) sequence and a consensus sequence A (CA). Normally, the poly(T) sequence is located at the 3'-end of the primer A' to initiate reverse transcription.
   (2) A primer B is annealed to or hybridize with the cDNA strand, in which the primer B comprises a consensus sequence B (CB) and a complementary sequence of the 3'-end overhang of the cDNA. In certain embodiments (e.g., when the method is used to construct a 5' transcriptome library), the primer B further comprises a unique molecular identifier (UMI). Subsequently, the nucleic acid fragment hybridized with or annealed to the primer B can be extended under the presence of a nucleic acid polymerase using the consensus sequence B and UMI sequence as templates, so that a complementary sequence of the consensus sequence B and a complementary sequence of the UMI sequence are added to the 3'-end of the cDNA strand, thereby generating a nucleic acid molecule that carries at the 5'-end the consensus sequence A, and carries at the 3'-end a complementary sequence of the consensus sequence B and a complementary sequence of the UMI molecule.

When the cDNA strand comprises at 3'-end an overhang of three cytosine nucleotides, the primer B can comprise GGG at its 3'-end. In addition, the nucleotides of the primer B can also be modified (e.g., the primer B can be modified to comprise a locked nucleic acid) to enhance the binding affinity for the complementary pairing between the primer B and the 3'-end overhang of the cDNA strand.

Without being limited by theory, various suitable nucleic acid polymerases (e.g., DNA polymerase or reverse transcriptase) can be used to perform the extension reaction, as long as they can extend the annealed or hybridized nucleic acid fragment (reverse transcription product) using the sequence or partial sequence of the primer B as a template. In certain exemplary embodiments, the annealed or hybridized nucleic acid fragment (reverse transcription product) can be extended using the same reverse transcriptase used in the aforementioned reverse transcription step.

In certain preferred embodiments, step (2) and step (1) are performed simultaneously.

In certain embodiments, the method optionally further comprises step (3): adding RNase H to digest the RNA strand in an RNA/cDNA hybrid to form a cDNA single strand.

In certain preferred embodiments, the method does not comprise step (3).

(4) An extension primer is used to perform an extension reaction using the cDNA single strand obtained in (3) as a template to obtain an extension product; the extension primer is capable of annealing to the whole or a part of a complementary sequence of the consensus sequence B, and initiating the extension reaction.

In certain embodiments, the extension primer is the same as the template switching oligo.

An exemplary structure comprising a complementary strand of the cDNA strand prepared by the above exemplary embodiment comprises: the consensus sequence B, the UMI sequence, a complementary sequence of the cDNA 3'-end overhang sequence, a complementary sequence of the cDNA sequence, a complementary sequence of the consensus sequence A.

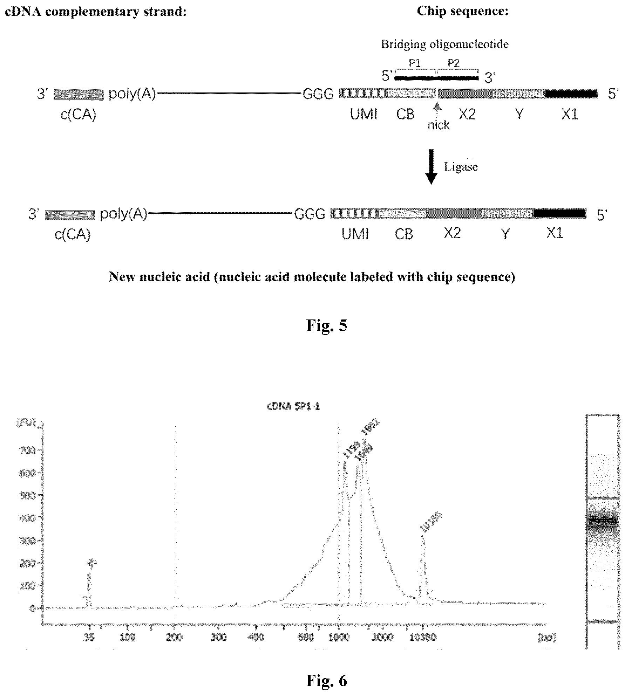

2. An exemplary embodiment, in which oligonucleotide probe (also called as chip sequence) is used to label the 5'-end of the complementary strand of the cDNA strand (that is, the 5'-end of the complementary strand of the cDNA strand is ligated to the 3'-end of the chip sequence) to generate a new nucleic acid molecule comprising the information of the chip sequence (i.e., a nucleic acid molecule labeled with the chip sequence), comprises the following steps (as shown in Fig. 5):
providing a bridging oligonucleotide, which comprises at 5'-end a sequence (a first region, P1) that is at least partially complementary to the consensus sequence B (CB), and comprises at 3'-end a sequence (a second region, P2) that is at least partially complementary to the consensus sequence X2.

In certain preferred embodiments, the P1 and P2 sequences in the bridging oligonucleotide are directly adjacent, and there is no intermediate nucleotides between them.

In certain preferred embodiments, the P1 sequence and P2 sequence each independently have a length of 20-100 nt (e.g., 20-70 nt).

The bridging oligonucleotide is annealed to or hybridized with the oligonucleotide probe and the complementary strand of the cDNA strand, and then the 5'-end of the complementary strand of the cDNA strand is ligated to the 3'-end of the chip sequence by a DNA ligase and/or a DNA polymerase to form a new nucleic acid molecule comprising the sequence information of the oligonucleotide probe (i.e., a nucleic acid molecule labeled with the oligonucleotide probe). In certain preferred embodiments, the DNA polymerase does not have 5' to 3' exonucleolytic activity or strand displacement activity.

An exemplary structure of the new nucleic acid molecule comprising the chip sequence information formed by the above exemplary embodiment comprises: the consensus sequence X1, the tag sequence Y, the consensus sequence X2, the consensus sequence B, the UMI sequence, a complementary sequence of the cDNA sequence, and a complementary sequence of the consensus sequence A.

In certain embodiments, the 3'-end overhang has at least 1, at least 2, at least 3, at least 4, at least 5, at least 6, at least 7, at least 8, at least 9, at least 10 or more nucleotides in length; in certain embodiments, the 3'-end overhang is a 3'-end overhang of 2-5 cytosine nucleotides (e.g., CCC overhang).

In some embodiments, in step (2) of the method, the biological sample is permeabilized before the preprocessing.

In certain embodiments, the biological sample is a tissue sample.

In certain embodiments, the tissue sample is a tissue section.

In certain embodiments, the tissue section is prepared from a fixed tissue, for example, a formalin-fixed paraffin-embedded (FFPE) tissue or deep-frozen tissue.

In certain embodiments, when the biological sample is in contact with the nucleic acid array, each cell of the biological sample individually occupies one or more microdots in the nucleic acid array (i.e., each cell is individually contacted with one or more microdot in the nucleic acid array).

In some embodiments, in step (2), the reverse transcription is performed by using a reverse transcriptase.

In certain embodiments, the reverse transcriptase has terminal deoxynucleotidyl transferase activity.

In certain embodiments, the reverse transcriptase is capable of synthesizing a cDNA strand using an RNA (e.g., mRNA) as a template and adding an overhang at the 3'-end of the cDNA strand.

In certain embodiments, the reverse transcriptase is capable of adding to the 3'-end of the cDNA strand an overhang with a length of at least 1, at least 2, at least 3, at least 4, at least 5, at least 6, at least 7, at least 8, at least 9, at least 10 or more nucleotides.

In certain embodiments, the reverse transcriptase is capable of adding to the 3'-end of the cDNA strand an overhang of 2-5 cytosine nucleotides (e.g., CCC overhang).

In certain embodiments, the reverse transcriptase is selected from the group consisting of M-MLV reverse transcriptase, HIV-1 reverse transcriptase, AMV reverse transcriptase, telomerase reverse transcriptase, and variants, modified products and derivatives thereof with the reverse transcription activity of the above-mentioned reverse transcriptases.

In certain embodiments, steps (2) and (3) of the method have one or more characteristics selected from the following:
(1) the primer A, primer A', primer B, and bridging oligonucleotide each independently comprise or consist of natural nucleotides (e.g., deoxyribonucleotides or ribonucleotides), modified nucleotides, non-natural nucleotides, or any combination thereof; in certain embodiments, the primer A and primer A' can initiate an extension reaction;
(2) the primer B comprises a modified nucleotide (e.g., locked nucleic acid); in certain embodiments, the primer B comprises one or more modified nucleotides (e.g., locked nucleic acid) at the 3'-end;
(3) the tag sequence A and tag sequence B each independently have a length of 5-200nt (e.g., 5-30nt, 6-15nt);
(4) the consensus sequence A and consensus sequence B each independently have a length of 10-200nt (e.g., 10-100nt, 20-100nt, 25-100nt, 5-10nt, 10-15nt, 15-20nt, 20-30nt, 30-40nt, 40-50nt, 50-100nt);
(5) the primer A, primer A', and primer B each independently have a length of 10-200 nt (e.g., 10-20nt, 20-30nt, 30-40nt, 40-50nt, 50-100nt, 100-150nt, 150-200nt);
(6) the first region and second region of the bridging oligonucleotide each independently have a length of 3-100nt (e.g., 3-10nt, 10-15nt, 15-20nt, 20-30nt, 30-40nt, 40-50nt, 50-100nt);
(7) the third region of the bridging oligonucleotide has a length of 0-100nt (e.g., 0-10nt, 10-15nt, 15-20nt, 20-30nt, 30-40nt, 40-50nt, 50-100nt);
(8) the bridging oligonucleotide has a length of 6-200nt (e.g., 20-70nt, 6-15nt, 15-20nt, 20-30nt, 30-40nt, 40-50nt, 50-100nt, 100-150nt, 150-200nt);
(9) the poly(T) sequence comprises at least 10, or at least 20 (e.g., at least 30) deoxythymidine residues;
(10) the random oligonucleotide sequence has a length of 5-200nt (e.g., 5-30nt, 6-15nt).

In certain embodiments, the method further comprises: (4) recovering and purifying the second nucleic acid molecule population.

In certain embodiments, in the method, the obtained second nucleic acid molecule population and/or complement thereof is used to construct a transcriptome library or for transcriptome sequencing.

In certain embodiments, the oligonucleotide probe in step (1) has one or more characteristics selected from the following:
(1) the consensus sequence X1, tag sequence Y, and consensus sequence X2 each independently comprises natural nucleotides (e.g., deoxyribonucleotides or ribonucleotides), modified nucleotides, non-natural nucleotides (e.g., peptide nucleic acid (PNA) or locked nucleic acid), or any combination thereof; in certain embodiments, the consensus sequence X2 has a free hydroxyl group (-OH) at the 3'-end;
(2) the consensus sequence X1, tag sequence Y and consensus sequence X2 each independently have a length of 2-100nt (e.g., 10-200nt, 10-100nt, 20-100nt, 25-100nt, 50-100nt, 5-30nt, 6-15nt, 5-10nt, 10-15nt, 15-20nt, 20-30nt, 30-40nt, 40-50nt);
(3) the oligonucleotide probes each independently have a length of 15-200nt (e.g., 15-20nt, 20-30nt, 30-40nt, 40-50nt, 50-100nt, 100-150nt, 150-200nt).

In certain embodiments, the oligonucleotide probe is coupled to the solid support via a linker.

In certain embodiments, the linker is a linking group capable of coupling with an activating group, and the surface of the solid support is modified with the activating group.

In certain embodiments, the linker comprises -SH, -DBCO, or -NHS.

In certain embodiments, the linker is -DBCO, and the surface of the solid support is modified with 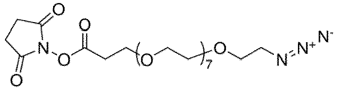 (Azido-dPEG^{®} 8-NHS ester).

In some embodiments, the nucleic acid array of step (1) has one or more characteristics selected from the following:
In certain embodiments, the oligonucleotide probes coupled to the same solid support have the same consensus sequence X1 and/or the same consensus sequence X2.

In certain embodiments, the consensus sequence X1 of the oligonucleotide probes comprises a cleavage site; in some embodiments, the cleavage site can be cleaved or broken by a method selected from nicking enzyme digestion, USER enzyme digestion, light-responsive excision, chemical excision or CRISPR-mediated excision.

In some embodiments, the nucleic acid array of step (1) is provided by the steps comprising:
(1) providing multiple kinds of carrier sequences, each kind of carrier sequence comprises at least one copy (e.g., multiple copies) of the carrier sequence, and the carrier sequence comprises in the direction from 5' to 3': a complementary sequence of the consensus sequence X2, a complementary sequence of the tag sequence Y, and an immobilization sequence; wherein, the complementary sequence of the tag sequence Y of each kind of carrier sequence is different from each other;
(2) attaching the multiple kinds of carrier sequences to the surface of a solid support (e.g., a chip);
(3) providing an immobilization primer, and using the carrier sequence as a template to perform a primer extension reaction to generate an extension product, so as to obtain the oligonucleotide probe; wherein the immobilization primer comprises the sequence of the consensus sequence X1, and is capable of annealing to the immobilization sequence of the carrier sequence and initiating an extension reaction; in some embodiments, the extension product in the direction from 5' to 3' comprises or consists of: the consensus sequence X1, the tag sequence Y, and the consensus sequence X2;
(4) linking the immobilization primer to the surface of the solid support; wherein steps (3) and (4) are performed in any order;
(5) optionally, the immobilization sequence of the carrier sequence further comprises a cleavage site, and the cleavage can be selected from nicking enzyme digestion, USER enzyme digestion, light-responsive excision, chemical excision or CRISPR-mediated excision; performing cleavage at the cleavage site comprised in the immobilization sequence of the carrier sequence to digest the carrier sequence, so that the extension product in step (3) is separated from the template (i.e., the carrier sequence) from which the extension product is generated, thereby linking the oligonucleotide probes to the surface of the solid support (e.g., chip).

In certain embodiments, each kind of carrier sequence is a DNB formed from a concatemer of multiple copies of the carrier sequence.

In certain embodiments, the multiple kinds of carrier sequences are provided in step (1) by the following steps:
(i) providing multiple kinds of carrier-template sequences, the carrier-template sequence comprises a complementary sequence of a carrier sequence;
(ii) using each kind of the carrier-template sequence as a template to perform a nucleic acid amplification reaction so as to obtain an amplification product of each kind of carrier-template sequence, wherein the amplification product comprises at least one copy (e.g., multiple copies) of the carrier sequence; in certain embodiments, rolling circle replication is performed to obtain a DNB formed from a concatemer of the carrier sequence.

In some embodiments, the solid support in step (1) has one or more characteristics selected from the following:
(1) the solid support is selected from the group consisting of latex bead, dextran bead, polystyrene surface, polypropylene surface, polyacrylamide gel, gold surface, glass surface, chip, sensor, electrode and silicon wafer; in some embodiments, the solid support is a chip;
(2) the solid support is planar, spherical or porous;
(3) the solid support can be used as a sequencing platform, such as a sequencing chip; in some embodiments, the solid support is a sequencing chip for Illumina, MGI or Thermo Fisher sequencing platform; and
(4) the solid support is capable of releasing the oligonucleotide probe spontaneously or upon exposure to one or more stimuli (e.g., temperature change, pH change, exposure to specific chemicals or phases, exposure to light, exposure to reducing agents, etc.).

Method of constructing nucleic acid molecule library

In another aspect, the present application also provides a method for constructing a nucleic acid molecule library, which comprises:
(a) generating a labeled nucleic acid molecule population according to the method as described above;
(b) randomly fragmenting the nucleic acid molecules in the labeled nucleic acid molecule population and adding an adapter; and
(c) optionally, amplifying and/or enriching the product of step (b);
thereby obtaining the nucleic acid molecule library.

In certain embodiments, the nucleic acid molecule library is used for sequencing, such as transcriptome sequencing, such as single cell transcriptome sequencing.

In certain embodiments, before performing step (b), the method further comprises step (pre-b): amplifying and/or enriching the labeled nucleic acid molecule population.

In certain embodiments, in step (pre-b), the labeled nucleic acid molecule population is subjected to a nucleic acid amplification reaction to generate an amplification product.

In certain embodiments, the nucleic acid amplification reaction is performed using at least a primer C and/or a primer D, wherein the primer C is capable of hybridizing with or annealing to a complementary sequence of the consensus sequence X1 or a complementary sequence of the 3'-end sequence of the consensus sequence X1, and initiating an extension reaction; the primer D is capable of hybridizing with or annealing to the nucleic acid molecules in the labeled nucleic acid molecule population, and initiating an extension reaction.

In certain embodiments, the nucleic acid amplification reaction in step (pre-b) is performed using a nucleic acid polymerase (e.g., a DNA polymerase; for example, a DNA polymerase with strand displacement activity and/or high fidelity).

In certain embodiments, in step (b), the nucleic acid molecule is randomly fragmented into fragments and an adapter is added to the fragments, using a transposase. In some embodiments, in step (b) of the method, the nucleic acid molecule obtained in the previous step is randomly fragmented into fragments and a first adapter and a second adapter are added to both ends of the fragments, respectively, using a transposase.

In certain embodiments, the transposase is selected from the group consisting of Tn5 transposase, MuA transposase, Sleeping Beauty transposase, Mariner transposase, Tn7 transposase, Tn10 transposase, Ty1 transposase, Tn552 transposase, as well as variants, modified products and derivatives thereof having the transposition activity of the above-mentioned transposases.

In certain embodiments, the transposase is Tn5 transposase.

In some embodiments, in step (c), the product of step (b) is amplified using at least a primer C' and/or a primer D', wherein the primer C' is capable of hybridizing with or annealing to the first adapter and initiating an extension reaction, and the primer D' is capable of hybridizing with or annealing to the second adapter and initiating an extension reaction.

In some embodiments, in step (c), at least the primer C and/or a primer D' is used to amplify the product of step (b); wherein the primer D' is capable of hybridizing with or annealing to the first adapter or the secondary adapter and initiating an extension reaction.

### Sequencing method

In another aspect, the present application also provides a method for sequencing a nucleic acid sample, which comprises:
(1) constructing a nucleic acid molecule library according to the method as described above; and
(2) sequencing the nucleic acid molecule library.

### Kit

In another aspect, the present application also provides a kit, which comprises:
(i) a nucleic acid array for labeling nucleic acids, which comprises a solid support, in which the solid support is coupled with multiple kinds of oligonucleotide probes; each kind of oligonucleotide probe comprises at least one copy; and, the oligonucleotide probe in the direction from 5' to 3' comprises or consists of: a consensus sequence X1, a tag sequence Y and a consensus sequence X2, wherein
   each kind of oligonucleotide probe has a different tag sequence Y, and the tag sequence Y has a nucleotide sequence unique to the position of the kind of oligonucleotide probe on the solid support;
(ii) a primer A, or a primer set comprising a primer A' and a primer B, or a primer set comprising a primer A and a primer B, wherein:
   the primer A comprises a consensus sequence A and a capture sequence A, in which the capture sequence A is capable of annealing to an RNA (e.g., mRNA) to be captured and initiating an extension reaction; in some embodiments, the consensus sequence A is located upstream of the capture sequence A (e.g., located at the 5'-end of the primer A);
   the primer A' comprises a capture sequence A, in which the capture sequence A is capable of annealing to an RNA (e.g., mRNA) to be captured and initiating an extension reaction;
   the primer B comprises a consensus sequence B, a complementary sequence of a 3'-end overhang, and optionally a tag sequence B; in certain embodiments, the complementary sequence of a 3'-end overhang is located at the 3'-end of the primer B; in certain embodiments, the consensus sequence B is located upstream of the complementary sequence of a 3'-end overhang (e.g., located at the 5'-end of the primer B); wherein the 3'-end overhang refers to one or more non-templated nucleotides comprised in the 3'-end of a cDNA strand generated by reverse transcription using the RNA captured by the capture sequence A of the primer A' as a template;
      and,
(iii) a bridging oligonucleotide, which comprises: a first region and a second region, and optionally a third region located between the first region and the second region, in which the first region is located upstream of the second region (e.g., located 5' of the second region); wherein,

the first region is capable of: (a) annealing to the whole or a part of the consensus sequence A of the primer A; or (b) annealing to the whole or a part of the consensus sequence B of the primer B;
the second region is capable of annealing to the whole or a part of the consensus sequence X2.

In certain embodiments, each kind of oligonucleotide probe comprises one copy.

In certain embodiments, each kind of oligonucleotide probe comprises multiple copies.

In certain embodiments, a region where each kind of oligonucleotide probe is coupled to the solid support is referred to as a microdot. It is easy to understand that when each kind of oligonucleotide probe comprises one copy, each microdot is coupled with one oligonucleotide probe, and the oligonucleotide probes of different microdots have different tag sequences Y; when each kind of oligonucleotide probe comprises multiple copies, each microdot is coupled with multiple oligonucleotide probes, and the oligonucleotide probes of the same microdot have the same tag sequence Y, and the oligonucleotide probes of different microdots have different tag sequences Y.

In certain embodiments, the solid support comprises a plurality of microdots, each microdot is coupled with one kind of oligonucleotide probe, and each kind of oligonucleotide probe may comprise one or more copies.

In certain embodiments, the solid support comprises a plurality of (e.g., at least 10, at least 10², at least 10³, at least 10⁴, at least 10⁵, at least 10⁶, at least 10⁷, at least 10⁸, or more) microdots; in certain embodiments, the solid support comprises at least 10⁴ (e.g., at least 10⁴, at least 10⁵, at least 10⁶, at least 10⁷, at least 10⁸, at least 10⁹, at least 10¹⁰, at least 10¹¹, or at least 10¹²) microdots/mm^{2.}

In some embodiments, the spacing between adjacent microdots is less than 100 µm, less than 50 µm, less than 10 µm, less than 5 µm, less than 1 µm, less than 0.5 µm, less than 0.1 µm, less than 0.05 µm, or less than 0.01 µm.

In certain embodiments, the microdots have a size (e.g., equivalent diameter) of less than 100 µm, less than 50 µm, less than 10 µm, less than 5 µm, less than 1 µm, less than 0.5 µm, less than 0.1 µm, less than 0.05 µm, or less than 0.01 µm.

In certain embodiments, the kit comprises: the nucleic acid array for labeling nucleic acids as described in (i), the primer A as described in (ii), and, the bridging oligonucleotide as described in (iii); wherein, the first region of the bridging oligonucleotide is capable of annealing to the whole or a part of the consensus sequence A of the primer A, and the second region of the bridging oligonucleotide is capable of annealing to the whole or a part of the consensus sequence X2.

In certain embodiments, the capture sequence A of the primer A is a random oligonucleotide sequence.

In certain embodiments, the capture sequence A of the primer A is a poly(T) sequence or a specific sequence targeting a target nucleic acid; in certain embodiments, the primer A further comprises a tag sequence A, such as a random oligonucleotide sequence; in certain embodiments, the capture sequence A is located at the 3'-end of the primer A, and the consensus sequence A is located upstream of the tag sequence A (e.g., located at the 5'-end of the primer A).

In certain embodiments, the primer A comprises a 5' phosphate at the 5'-end.

In certain embodiments, the kit comprises: the nucleic acid array for labeling nucleic acids as described in (i), the primer set comprising the primer A' and primer B as described in (ii), and, the bridging oligonucleotide as described in (iii); wherein the first region of the bridging oligonucleotide is capable of annealing to the whole or a part to the consensus sequence B of the primer B, and the second region of the bridging oligonucleotide is capable of annealing to the whole or a part of the consensus sequence X2.

In certain embodiments, the capture sequence A of the primer A' is a random oligonucleotide sequence.

In certain embodiments, the capture sequence A of the primer A' is a poly(T) sequence or a specific sequence targeting a target nucleic acid; in certain embodiments, the primer A' further comprises a tag sequence A, and a consensus sequence A; in certain embodiments, the capture sequence A is located at the 3'-end of the primer A'; in certain embodiments, the consensus sequence A is located upstream of the capture sequence A (e.g., located at the 5'-end of the primer A').

In certain embodiments, the primer B comprises the consensus sequence B, a complementary sequence of the 3'-end overhang, and the tag sequence B.

In certain embodiments, the kit further comprises a primer B', in which the primer B' is capable of annealing to the whole or a part of a complementary sequence of the consensus sequence B and initiating an extension reaction.

In certain embodiments, the primer B or primer B' comprises a 5' phosphate at the 5'-end.

In certain embodiments, the primer B comprises a modified nucleotide (e.g., a locked nucleic acid); in certain embodiments, the primer B comprises one or more modified nucleotides (e.g., one or more locked nucleic acid) at the 3'-end.

In certain embodiments, the kit comprises: the nucleic acid array for labeling nucleic acids as described in (i), the primer set comprising the primer A and primer B as described in (ii), and, the bridging oligonucleotide described in (iii); wherein the first region of the bridging oligonucleotide is capable of annealing to the whole or a part of the consensus sequence A of the primer A, and the second region of the bridging oligonucleotide is capable of annealing to the whole or a part of the consensus sequence X2.

In certain embodiments, the capture sequence A of the primer A is a random oligonucleotide sequence.

In certain embodiments, the capture sequence A of the primer A is a poly(T) sequence or a specific sequence targeting a target nucleic acid; in certain embodiments, the primer A further comprises a tag sequence A, such as a random oligonucleotide sequence; in certain embodiments, the capture sequence A is located at the 3'-end of the primer A, and the consensus sequence A is located upstream of the tag sequence A (e.g., located at the 5'-end of the primer A).

In certain embodiments, the primer A comprises a 5' phosphate at the 5'-end.

In certain embodiments, the primer B comprises a modified nucleotide (e.g., locked nucleic acid). In certain embodiments, the primer B comprises one or more modified nucleotides (e.g., locked nucleic acid) at the 3'-end.

In certain embodiments, the kit has one or more characteristics selected from the following:
(1) the oligonucleotide probe, primer A, primer A', primer B, primer B', and bridging oligonucleotide each independently comprise or consist of natural nucleotides (e.g., deoxyribonucleotides or ribonucleotides), modified nucleotides, non-natural nucleotides, or any combination thereof; in certain embodiments, the primer A, primer A', and primer B' are capable of initiating an extension reaction; in certain embodiments, the consensus sequence X2 has a free hydroxyl group (-OH) at the 3'-end;
(2) the oligonucleotide probes each independently have a length of 15-300nt (e.g., 15-200nt, 15-20nt, 20-30nt, 30-40nt, 40-50nt, 50-100nt, 100-150nt, 150-200nt);
(3) the primer A, primer A', primer B, and primer B' each independently have a length of 10-200 nt (e.g., 10-20 nt, 20-30 nt, 30-40 nt, 40-50 nt, 50-100 nt, 100-150 nt, 150-200 nt);
(4) the bridging oligonucleotide has a length of 6-200nt (e.g., 20-70nt, 6-15nt, 15-20nt, 20-30nt, 30-40nt, 40-50nt, 50-100nt, 100-150 nt, 150-200 nt);
(5) the oligonucleotide probes coupled to the same solid support have the same consensus sequence X1 and/or the same consensus sequence X2;
(6) the consensus sequence X1 of the oligonucleotide probes comprises a cleavage site; in some embodiments, the cleavage site can be cleaved or broken by a method selected from nicking enzyme digestion, USER enzyme digestion, light-responsive excision, chemical excision or CRISPR-mediated excision.

In certain embodiments, the kit further comprises a reverse transcriptase, a nucleic acid ligase, a nucleic acid polymerase and/or a transposase.

In certain embodiments, the reverse transcriptase has terminal deoxynucleotidyl transferase activity; in certain embodiments, the reverse transcriptase is capable of synthesizing a cDNA strand using an RNA (e.g., mRNA) as a template, and adding a 3'-end overhang to the 3'-end of the cDNA strand; in certain embodiments, the reverse transcriptase is capable of adding to the 3'-end of the cDNA strand an overhang having a length of at least 1, at least 2, at least 3, at least 4, at least 5, at least 6, at least 7, at least 8, at least 9, at least 10 or more nucleotides; in certain embodiments, the reverse transcriptase is capable of adding to the 3'-end of the cDNA strand an overhang of 2-5 cytosine nucleotides (e.g., CCC overhang); in certain embodiments, the reverse transcriptase is selected from the group consisting of M-MLV reverse transcriptase, HIV-1 reverse transcriptase, AMV reverse transcriptase, telomerase reverse transcriptase, and variants, modified products and derivatives thereof having the reverse transcription activity of the above-mentioned reverse transcriptases.

In certain embodiments, the nucleic acid polymerase does not have 5' to 3' exonucleolytic activity or strand displacement activity.

In certain embodiments, the transposase is selected from the group consisting of Tn5 transposase, MuA transposase, Sleeping Beauty transposase, Mariner transposase, Tn7 transposase, Tn10 transposase, Ty1 transposase, Tn552 transposase, as well as variants, modified products and derivatives thereof having the transposition activity of the above-mentioned transposases.

In certain embodiments, the kit further comprises: the primer C, the primer D, the primer C' and/or the primer D'. For example, the kit further comprises the primer C, the primer D and the primer D'. For example, the kit further comprises the primer C, the primer D, the primer C' and the primer D'.

In certain embodiments, the kit further comprises: reagents for nucleic acid hybridization, reagents for nucleic acid extension, reagents for nucleic acid amplification, reagents for recovering or purifying nucleic acids, reagents for constructing transcriptome sequencing library, reagents for sequencing (e.g., second- or third-generation sequencing), or any combination thereof.

### Use

In another aspect, the present application also provides a use of the method for generating a labeled nucleic acid molecule population as described above or the kit as described above for constructing a nucleic acid molecule library or for performing transcriptome sequencing.

### Definition of Terms

In the present application, unless otherwise stated, scientific and technical terms used herein have the meanings commonly understood by those skilled in the art. Moreover, the operating steps of molecular biology, biochemistry, nucleic acid chemistry, cell culture, etc., as used herein are all routine steps widely used in the corresponding fields. Meanwhile, in order to better understand the present application, definitions and explanations of relevant terms are provided below.

When the terms "e.g.," "for example," "such as," "comprise," "include," or variations thereof are used herein, these terms will not be considered limiting terms and will instead be interpreted to mean "but not limited" or "without limitation."

Unless otherwise indicated herein or clearly contradicted by context, the terms "a" and "an" as well as "the" and similar referents in the context of describing the present application (especially in the context of the following claims) are to be construed to cover singular and plural.

As used in this article, "DNB" (DNA nanoball) is a typical RCA (rolling circle amplification) product, which has the characteristics of RCA products. Wherein, the RCA product is a single-stranded DNA sequence with multiple copies, which can form a similar "spherical" structure due to the interaction between the bases comprised in the DNA. Typically, a library molecule is circularized to form a single-stranded circular DNA, and subsequently the single-stranded circular DNA can be amplified by multiple orders of magnitude using the rolling circle amplification technology, thereby generating an amplification product called DNB.

As used herein, a "nucleic acid molecule population" refers to a population or collection of nucleic acid molecules, for example, nucleic acid molecules derived directly or indirectly from a target nucleic acid molecule (e.g., a double-stranded DNA, an RNA/cDNA hybrid, a single-stranded DNA, or a single-stranded RNA). In some embodiments, a nucleic acid molecule population comprises a nucleic acid molecule library, and the nucleic acid molecule library comprises sequences that are qualitatively and/or quantitatively representative of a target nucleic acid molecule sequence. In other embodiments, the nucleic acid molecule population comprises a subset of the nucleic acid molecule library.

As used herein, a "nucleic acid molecule library" refers to a collection or population of labeled nucleic acid molecules (e.g., labeled double-stranded DNA, labeled RNA/cDNA hybrid, labeled single-stranded DNA, or labeled single-stranded RNA) or fragments thereof that are generated directly or indirectly from a target nucleic acid molecule, wherein the combination of labeled nucleic acid molecules or fragments thereof in the collection or population is shown to be qualitatively and/or quantitatively representative of the sequence of a target nucleic acid molecule sequence from which the labeled nucleic acid molecules are generated. In certain embodiments, the nucleic acid molecule library is a sequencing library. In certain embodiments, the nucleic acid molecule library can be used to construct a sequencing library.

As used herein, a "cDNA" or "cDNA strand" refers to a "complementary DNA" synthesized by extension using at least a portion of an RNA molecule of interest as a template via a primer that anneals to the RNA molecule of interest under catalysis of an RNA-dependent DNA polymerase or reverse transcriptase (this process is also called "reverse transcription"). The synthesized cDNA molecule is "homologous" to or "complementary" to or "base pairs" with or "forms a complex" with at least a portion of the template.

As used herein, the term "upstream" is used to describe the relative positional relationship of two nucleic acid sequences (or two nucleic acid molecules) and has a meaning commonly understood by those skilled in the art. For example, the expression "a nucleic acid sequence is located upstream of another nucleic acid sequence" means that, when aligned in the 5' to 3' direction, the former is located at a more forward position (i.e., a position closer to the 5'-end) than the latter. As used herein, the term "downstream" has the opposite meaning to "upstream."

As used herein, a "tag sequence Y", "tag sequence A", "tag sequence B", "consensus sequence X1", "consensus sequence X2", "consensus sequence A", "consensus sequence B", etc., refers to an oligonucleotide having non-target nucleic acid components that provide a means of identification, recognition, and/or molecular manipulation or biochemical manipulation (e.g., by providing a site for annealing an oligonucleotide, the oligonucleotide being, for example, a primer for DNA polymerase extension or an oligonucleotide for capture reaction or ligation reaction) for a nucleic acid molecule ligated thereto or a derivative product of the nucleic acid molecule ligated thereto (e.g., a complementary fragment of the nucleic acid molecule, a short fragment of the nucleic acid molecule, etc.). The oligonucleotide may consist of at least two (preferably about 6 to 100, but there is no definite limit to the length of the oligonucleotide, and the exact size depends on many factors, while these factors in turn depend on the final function or use of the oligonucleotide) nucleotides, and can also be composed of multiple oligonucleotide segments in a continuous or non-continuous arrangement. The oligonucleotide sequence may be unique to each nucleic acid molecule to which it is ligated, or may be unique to a certain type of nucleic acid molecule to which it is ligated. The oligonucleotide sequence may be reversibly or irreversibly ligated to a polynucleotide sequence to be "labeled" by any method including ligation, hybridization or other methods. The process of ligating the oligonucleotide sequence to a nucleic acid molecule is sometimes referred to herein as "labeling", and a nucleic acid molecule that undergoes the addition of a label or comprises a label sequence is called a "labeled nucleic acid molecule" or "tagged nucleic acid molecule."

For various reasons, the nucleic acid or polynucleotide of the present application (e.g., "tag sequence Y", "tag sequence A", "tag sequence B", "consensus sequence X1", "consensus sequence X2", "consensus sequence A" ", "consensus sequence B", "primer A", "primer A'", "primer B", "primer B'", "primer C", "primer D", "primer D'", "bridging oligonucleotide", etc.) may comprise one or more modified nucleic acid bases, sugar moieties, or internucleoside linkages. For example, some reasons for using nucleic acids or polynucleotides comprising modified bases, sugar moieties, or internucleoside linkages include, but are not limited to: (1) changes in Tm; (2) changes in the susceptibility of polynucleotide to one or more nucleases; (3) providing a moiety for linking a label; (4) providing a label or label quencher; or (5) providing a moiety such as biotin for attaching another molecule in solution or bound to a surface. For example, in some embodiments, oligonucleotides such as primers can be synthesized such that the random portions comprise one or more nucleic acid analogs with constrained conformation, including, but not limited to, one or more ribonucleic acid analogs in which ribose ring is "locked" by the methylene bridge that links the 2'-O atom to the 4'-C atom; these modified nucleotides result in an increase in the Tm, or melting temperature, of each molecule by about 2 degrees Celsius to about 8 degrees Celsius. For example, in some embodiments in which an oligonucleotide primer comprising ribonucleotides is used, one indicator of using a modified nucleotide in the method may be that the oligonucleotide comprising the modified nucleotide may be digested by a single-strand specific RNase.

In the methods of the present application, for example, the nucleic acid bases in single nucleotides at one or more positions in a polynucleotide or oligonucleotide may comprise guanine, adenine, uracil, thymine or cytosine; or optionally, one or more of the nucleic acid bases may comprise modified bases such as, but not limited to, xanthine, allylamino-uracil, allylamino-thymine nucleoside, hypoxanthine, 2-aminoadenine, 5-propynyluracil, 5-propynylcytosine, 4-thiouracil, 6-thioguanine, azauracil, deazauracil, thymine nucleoside, cytosine, adenine or guanine. Furthermore, they may comprise nucleic acid bases derivatized with the following moieties: biotin moiety, digoxigenin moiety, fluorescent or chemiluminescent moiety, quenching moiety or some other moieties. The present application is not limited to the listed nucleic acid bases; the list given illustrates examples of a wide range of bases that may be used in the methods of the present application.

With respect to the nucleic acids or polynucleotides of the present application, one or more of the sugar moieties may comprise 2'-deoxyribose, or optionally, one or more of the sugar moieties may comprise some other sugar moieties, such as, but not limited to: ribose or 2'-fluoro-2'-deoxyribose or 2'-O-methyl-ribose that possesses resistance to some nucleases, or 2'-amino-2'-deoxyribose or 2'-azido-2'-deoxyribose that is labeled by reaction with a visible, fluorescent, infrared fluorescent or other detectable dye or a chemical substance with an electrophilic, photoreactive, alkynyl or other reactive chemical moiety.

The internucleoside linkages of the nucleic acids or polynucleotides of the present application may be phosphodiester linkages, or optionally, one or more of the internucleoside linkages may comprise modified linkages such as, but not limited to: phosphorothioate, phosphorodithioate, phosphoroselenate, or phosphorodiselenate linkages, which are resistant to some nucleases.

As used herein, the term "terminal deoxynucleotidyl transferase activity" refers to an ability to catalyze the template-independent addition (or "tailing") of one or more deoxyribonucleoside triphosphates (dNTPs) or single dideoxyribonucleoside triphosphates to the 3'-end of cDNA. Examples of reverse transcriptases with terminal deoxynucleotidyl transferase activity comprise, but are not limited to, M-MLV reverse transcriptase, HIV-1 reverse transcriptase, AMV reverse transcriptase, telomerase reverse transcriptase, and variants, modified products and derivatives thereof with the reverse transcription activity and terminal deoxynucleotidyl transferase activity of the reverse transcriptases. The reverse transcriptases have or do not have RNase activity (especially RNase H activity). In preferred embodiments, the reverse transcriptases used for the reverse transcription of RNA to generate cDNA do not have RNase activity (especially RNase H activity). Therefore, in a preferred embodiment, the reverse transcriptase used for the reverse transcription of RNA to generate cDNA has terminal deoxynucleotidyl transferase activity, and does not have RNase activity (especially RNase H activity).

As used herein, a nucleic acid polymerase with "strand displacement activity" refers to a nucleic acid polymerase that, during the process of extending a new nucleic acid strand, if it encounters a downstream nucleic acid strand complementary to the template strand, can continue the extension reaction and replace (rather than degrade) the nucleic acid strand that is complementary to the template strand.

As used herein, a nucleic acid polymerase having "5' to 3' exonucleolytic activity" refers to a nucleic acid polymerase that can catalyze the hydrolysis of 3,5-phosphodiester bonds in the order of 5'-end to 3'-end of a polynucleotide, thereby degrading nucleotides.

As used herein, a nucleic acid polymerase (or DNA polymerase) with "high fidelity" refers to a nucleic acid polymerase (or DNA polymerase) that has a lower probability of introducing erroneous nucleotides (i.e., an error rate) during the amplification of nucleic acids than the wild-type Taq enzyme (e.g., the Taq enzyme whose sequence is shown in UniProt Accession: P19821.1).

As used herein, the terms "annealed," "annealing," "anneal," "hybridized," or "hybridizing" and the like refer to the formation of complex between nucleotide sequences having sufficient complementarity to form complex via Watson-Crick base pairing. For the purposes of the present application, nucleic acid sequences that "are complementary" or "hybridize" or "anneal" to each other should be capable of forming a sufficiently stable "hybrid" or "complex" for the intended purpose. It is not required that every nucleic acid base within the sequence displayed by a nucleic acid molecule is capable of base pairing or pairing or complexing with every nucleic acid base within the sequence displayed by another nucleic acid molecule such that both nucleic acid molecules or corresponding sequences displayed therein "are complementary" or "anneal" or "hybridize" to each other. As used herein, the term "complementary" or "complementarity" is used when referring to sequences of nucleotides that are related by the rules of base pairing. For example, the sequence 5'-A-G-T-3' is complementary to the sequence 3'-T-C-A-5'. Complementarity can be "partial", in which only some of the nucleic acid bases match according to the rules of base pairing. Optionally, there may be "complete" or "total" complementarity between nucleic acids. The degree of complementarity between nucleic acid strands has a significant impact on the efficiency and strength of hybridization between the nucleic acid strands. The degree of complementarity is particularly important in amplification reactions and detection methods that rely on hybridization of nucleic acids. The term "homology" refers to the degree of complementarity of one nucleic acid sequence to another nucleic acid sequence. There may be partial homology (i.e., complementarity) or complete homology (i.e., complementarity). A partially complementary sequence is a sequence that at least partially inhibits hybridization of a fully complementary sequence to a target nucleic acid and is referred to using a functional term "substantially homologous". Inhibition of hybridization of a fully complementary sequence to a target sequence can be tested under low stringency conditions using hybridization assays (e.g., Southern blotting or Northern blotting, hybridization in solution, etc.). Substantially homologous sequences or probes will compete in or inhibit binding (i.e., hybridization) of fully homologous sequences to the target under conditions of low stringency. This is not to say that low stringency conditions are conditions that allow for nonspecific binding; low stringency conditions require that the two sequences bind to each other via a specific (i.e., selective) interaction. The absence of non-specific binding can be tested by using a second target that lacks complementarity or has only a low degree of complementarity (e.g., less than about 30% complementarity). In cases where specific binding is low or absent, the probe will not hybridize to the nucleic acid target. When used in reference to a double-stranded nucleic acid sequence such as a cDNA or genomic clone, the term "substantially homologous" means it is any oligonucleotide or probe that can hybridize to one or both strands of the double-stranded nucleic acid sequence under the low stringency conditions described herein. As used herein, the terms "annealing" or "hybridization" are used when referring to the pairing of complementary nucleic acid strands. Hybridization and hybridization strength (i.e., strength of association between nucleic acid strands) are affected by many factors known in the art, including the degree of complementarity between nucleic acids, including the stringency of conditions affected by factors such as salt concentration, the Tm (melting temperature) to form a hybrid, the presence of other components (e.g., the presence or absence of polyethylene glycol or betaine), the molar concentration of hybridized strands, and the GC content of nucleic acid strands.

As described herein, the solid support is capable of releasing the oligonucleotide probe spontaneously or upon exposure to one or more stimuli (e.g., temperature changes, pH changes, exposure to specific chemicals or phases, exposure to light, reducing agents, etc.). It will be appreciated that the oligonucleotide probe can be released by cleavage of the bond between the oligonucleotide probe and the solid support, or degradation of the solid support itself, or both, and the oligonucleotide probe allows or is capable of being approached by other reagents.

Adding multiple types of labile bonds to the solid support enables the capability of the solid support to respond to different stimuli. Each type of labile bond can be sensitive to an associated stimulus (e.g., chemical stimulus, light, temperature, etc.) such that the release of a substance attached to the solid support through each labile bond can be controlled by applying an appropriate stimulus. In addition to thermally cleavable bonds, disulfide bonds, and UV-sensitive bonds, other non-limiting examples of labile bonds that can be coupled to the solid support comprise ester bonds (e.g., ester bonds that can be cleaved with acids, bases, or hydroxylamine), ortho diol bonds (e.g., ortho diol bonds that can be cleaved by sodium periodate), Diels-Alder bonds (e.g., Diels-Alder bonds that can be cleaved thermally), sulfone bonds (e.g., sulfone bonds that can be cleaved by alkali), silicyl ether bonds (e.g., silicyl ether bonds that can be cleaved by acids), glycosidic bonds (e.g., glycosidic bonds that can be cleaved by amylase), peptide bonds (e.g., peptide bonds that can be cleaved by proteases), or phosphodiester bonds (e.g., phosphodiester bonds that can be cleaved by nucleases (e.g., DNA enzyme)).

In addition to or as an alternative to the cleavable bonds between the solid support and the oligonucleotide described above, the solid support can be degradable, destructible or soluble spontaneously or upon exposure to one or more stimuli (e.g., temperature changes, pH changes, exposure to specific chemical substances or phases, exposure to light, exposure to reducing agents, etc.). In some cases, the solid support may be soluble such that the material components of the solid support dissolve upon exposure to specific chemicals or environmental changes (e.g., changes in temperature or changes in pH). In some cases, the solid support may degrade or dissolve under elevated temperatures and/or alkaline conditions. In some cases, the solid support may be thermally degradable such that the solid support degrades when exposed to appropriate temperature changes (e.g., heating). Degradation or dissolution of the solid support bound with a substance (e.g., an oligonucleotide probe) can result in the release of the substance from the solid support.

As used herein, the terms "transposase" and "reverse transcriptase" and "nucleic acid polymerase" refer to a protein molecule or an aggregate of protein molecules responsible for catalyzing specific chemical and biological reactions. In general, the methods, compositions or kits of the present application are not limited to the use of a specific transposase, reverse transcriptase or nucleic acid polymerase from a specific source. Rather, the methods, compositions, or kits of the present application may comprise any transposases, reverse transcriptases, or nucleic acid polymerases from any sources that have equivalent enzymatic activity to the specific enzymes of the specific methods, compositions, or kits disclosed herein. Furthermore, the methods of the present application also comprise the following embodiments: wherein any one specific enzyme provided and used in the steps of the methods is replaced by a combination of two or more enzymes, when the two or more enzymes are used in combination, whether used separately in a stepwise manner or together simultaneously, the reaction mixtures produce the same results as would be obtained using that specific enzyme. The methods, buffers, and reaction conditions provided herein, including those in the Examples, are currently preferred for embodiments of the methods, compositions, and kits of the present application. However, other enzyme storage buffers, reaction buffers, and reaction conditions can be used for some of the enzymes of the present application are known in the art and may also be suitable for use in the present application and are comprised herein.

### Beneficial effects of the present application

The present application provides a new method for generating a labeled nucleic acid molecule population, as well as a method for constructing a nucleic acid molecule library based on the method and performing a high-throughput sequencing, thereby achieving high-precision subcellular-level spatial positioning of samples. The method of the present application has one or more beneficial technical effects selected from the following:
(1) The probes of traditional nucleic acid arrays (e.g., chips) used for spatial transcriptome sequencing comprise fixed capture sequences. Usually a specific capture sequence can only capture the specific target nucleic acid molecule corresponding to it. For example, when the capture sequence is a poly (T), it correspondingly captures a target nucleic acid molecule comprising poly(A). If the target nucleic acid molecule changes, the probe sequence comprising the capture sequence needs to be changed accordingly, that is, the entire nucleic acid array (e.g., a chip) needs to be changed, which is costly and inefficient in practical applications. The nucleic acid array (e.g., a chip) of the present application does not comprise a capture sequence, and the capture sequence exists in reverse transcription primer that is independent of the nucleic acid array (that is, the capture sequence and the probe are independent of each other). After the capture sequence captures the target nucleic acid molecule, it ligates to the probe via a bridging oligonucleotide.

Therefore, the present application can design corresponding capture sequences for different target nucleic acid molecules without changing the probe sequence (that is, without changing the nucleic acid array (e.g., a chip)), and achieve the capture of different target nucleic acid molecules by changing the capture sequences and the bridging oligonucleotides.

(2) Traditional spatial transcriptome methods all use a poly(T) as a capture sequence and cannot capture an RNA without a poly(A) tail, while the present application can achieve the capture of target nucleic acid molecules without poly(A) tails by replacing the poly(T) in the capture sequence with a continuous random sequence group (e.g., a random primer sequence, such as N6, N8, etc.), and, the continuous random sequence group can also serve as a unique molecular identifier (UMI) sequence at the same time.

(3) Traditional nucleic acid arrays (e.g., chips) used for spatial transcriptome sequencing have fixed capture probes. Generally, tissue permeabilization is performed first to release intracellular RNA. If permeabilization is excessive, RNA will spread to adjacent cells, even the periphery of the tissue sample, and is captured by the probes, making it impossible to achieve in-situ capture of mRNA. If permeabilization is incomplete, the capture efficiency of mRNA will be affected. With the method of the present application, the nucleic acid array (e.g., a chip) does not comprise a capture sequence (the nucleic acid array comprises spatial information and does not comprise a capture sequence), and the purpose of tissue permeabilization is to allow the reverse transcription primer to enter the cell and hybridize with the mRNA in situ, without the need for intense permeabilization reagent treatment, thereby reducing sample spread.

The preferred embodiments of the present application are described in detail below in conjunction with the accompanying drawings and examples, but those skilled in the art will understand that the following drawings and examples are only used to illustrate the present application and do not limit the scope of the present application. The various objects and advantageous aspects of the present application will become apparent to those skilled in the art from the accompanying drawings and the following detailed description of preferred embodiments.

### Brief Description of the Drawings

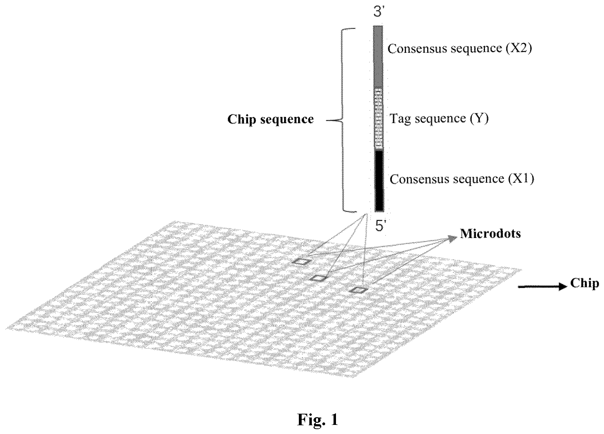

Fig. 1 shows an exemplary structure of a chip used for capturing and labeling nucleic acid molecules in the present application, which comprises: a chip and an oligonucleotide probe (also called a chip sequence) coupled to the chip. Each kind of oligonucleotide probe comprises a tag sequence Y corresponding to its position on the chip, and a region where each kind of oligonucleotide probe is coupled to the chip can be called a microdot. Each kind of oligonucleotide probe may comprise single copy or multiple copies.
Fig. 2 shows an exemplary scheme for preparing a cDNA strand using an RNA (e.g., mRNA) in a sample as a template, as well as an exemplary structure of the cDNA strand. CA: consensus sequence A; CB: consensus sequence B.
Fig. 3 shows an exemplary scheme for generating a new nucleic acid molecule comprising information of a chip sequence (i.e., a chip sequence-labeled nucleic acid molecule) by labeling the 5'-end of a cDNA strand with the chip sequence (i.e., ligating the 5'-end of the cDNA strand to the 3'-end of the chip sequence), and an exemplary structure of the new nucleic acid molecule comprising the chip sequence information. CA: consensus sequence A; CB: consensus sequence B; X1: consensus sequence X1, Y: tag sequence Y; X2: consensus sequence X2.
Fig. 4 shows an exemplary scheme for preparing a complementary strand of a cDNA strand using an RNA (e.g., mRNA) in a sample as a template, as well as an exemplary structure of the complementary strand of the cDNA strand. CA: consensus sequence A; CB: consensus sequence B; EP: extension primer.
Fig. 5 shows an exemplary scheme for generating a new nucleic acid molecule comprising information of a chip sequence (i.e., a chip sequence-labeled nucleic acid molecule), by labeling the 5'-end of a complementary strand of a cDNA strand with the chip sequence (i.e., ligating the 5'-end of the complementary strand of the cDNA strand to the 3'-end of the chip sequence), and an exemplary structure of the new nucleic acid molecule comprising the chip sequence information. CA: consensus sequence A; CB: consensus sequence B; X1: consensus sequence X1, Y: tag sequence Y; X2: consensus sequence X2.
Fig. 6 shows the length distribution of the cDNA amplification products prepared in Example 2.
Fig. 7 shows the mouse brain gene expression map obtained by the method of the present application.
Fig. 8 shows a zoomed mouse brain gene expression map obtained by the method of the present application.

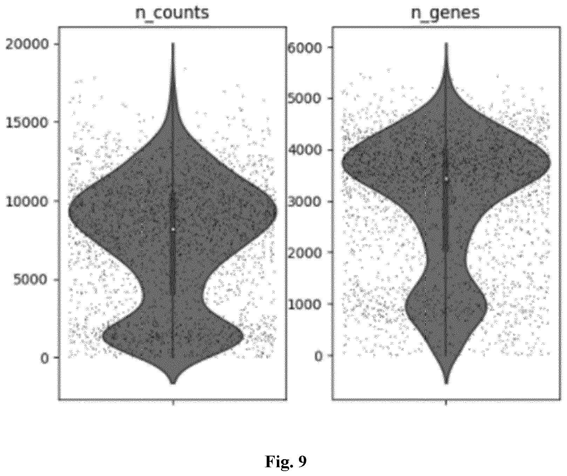

Fig. 9 shows the number of genes captured by the method of the present application and the UMI analysis results.

### Specific Models for Carrying Out the present application

The present application will now be described with reference to the following examples which are intended to illustrate (but not to limit) the present application. Unless otherwise indicated, the experiments and methods described in the examples were performed essentially according to conventional methods well known in the art and described in various references. In addition, if the specific conditions are not specified in the examples, the conventional conditions or the conditions recommended by the manufacturer should be followed. If the manufacturer of the reagents or instruments used was not indicated, they were all conventional products that could be purchased commercially. Those skilled in the art will appreciate that the examples describe the present application by way of example and are not intended to limit the scope sought to be protected by the present application. All publications and other references mentioned herein are incorporated by reference in their entirety.

### Sequence Information

Information on some of the sequences covered by the present application is provided in Table 1 below.

**Table 1: Sequence information**

| SEQ ID NO | Description | Sequence information |
|---|---|---|
| 1 | Nucleotide sequence of DNA library molecule | p-GAACGACATGGCTACGATCCGACTTNN NNNNNNNNNNNNNNNAAGTCGGAGGCCAAGC GGTCTTAGGAAGACAANNNNNNNNNNNNNN NNNNNNNNNNNCTGATAAGGTCGCCATGCC |
| | | TCTCAGTACGTCAGCAGTTNNNNNNNNNNNNNN NNNNNNCAACTCCTTGGCTCACA |
| 2 | Nucleotide sequence of DNB primer | GCCATGTCGTTCGGAACCGAGTGT |
| 3 | Nucleotide sequence of chip sequence synthesis primer | CTGCTGACGTACTGAGAGGCATGGCGACCTT ATCAG |
| 4 | Consensus sequence X1 | CTGCTGACGTACTGAGAGGCATGGCGACCTT ATCAG |
| 5 | Consensus sequence X2 | TTGTCTTCCTAAG |
| 6 | Nucleotide sequence of polyT primer | p-ACCGCTTGGCCTCCGNNNNNNNNNNTTTTT TTTTTTTTTTTTTTTTTTTTTTTTTV |
| 7 | TSO sequence | AAGCAGTGGTATCAACGCAGAGTACATrGrG +G |
| 8 | Chip sequence | CTGCTGACGTACTGAGAGGCATGGCGACCTT ATCAGNNNNNNNNNNNNNNNNNNNNNNNN NTTGTCTTCCTAAG |
| 9 | Bridging oligonucleotide sequence | AGGCCAAGCGGTCTTAGGAAGACA |
| 10 | Sequence of cDNA amplification primer 1 | CTGCTGACGTACTGAGAGGCATG |
| 11 | Sequence of cDNA amplification primer 2 | AAGCAGTGGTATCAACGCAGAGTAC |
| 12 | Library construction primer 1 | p-CTGCTGACGTACTGAGAGGC*A*T |
| 13 | Library construction primer 2 | GAGACGTTCTCGACTCAGAAGATG |
| 14 | Primer sequence to prepare DNB for sequencing | GGCCTCCGACTTGAGACGTTCTCG |

| | | |
|---|---|---|
| Note: "r" indicates that the nucleotide at the 3' adjacent position is ribonucleotide; "+" indicates that the nucleotide at the 3' adjacent position comprises LNA (locked nucleotide); "*" indicates phosphorothioate modification; "p" indicates phosphorylation modification. | | |

### Example 1: Preparation of capture chip

1. Sequences of DNA library molecules comprising the position information of a chip were designed, which comprised from 5' to 3': consensus sequence X1 (X1), tag sequence (Y) and consensus sequence X2 (X2). A typical nucleotide sequence of a DNA library molecule was shown in SEQ ID NO: 1. Beijing Liuhe BGI Co., Ltd. was entrusted to synthesize the DNA library molecules.
2. Amplification and loading of library molecules
   (1) A DNBSEQ sequencing kit (purchased from MGI, Cat. No. 1000019840) was used to prepare DNA nano balls (DNBs). Specific embodiment was briefly described below.

Briefly, 40 µL of the reaction system as shown in Table 2 was prepared. The reaction system was placed in a PCR machine and the reaction was performed according to the following reaction conditions: 95°C for 3 minutes, 40°C for 3 minutes. After the reaction was completed, the reaction product was placed on ice, added with 40 µL of mixed enzyme I and 2 µL of mixed enzyme II (from DNBSEQ sequencing kit), 1 µL of ATP (100 mM stock solution, obtained from Thermo Fisher), and 0.1 µL of T4 ligase (obtained from NEB, Cat. No.: M0202S). After mixing well, the above reaction system was placed in a PCR machine and reacted at 30°C for 20 minutes to generate DNBs.

**Table 2: Reaction system for preparing DNBs**

| Ingredient | Volume (µL) | Final concentration |
|---|---|---|
| DNA library molecule | X (80 fmol) | - |
| 10X phi29 buffer | 4 | 1X |
| 10 µM DNB primer (SEQ ID NO:2; synthesized by Liuhe BGI) | 4 | 1 µM |
| H₂O | 32-x | - |

(2) Subsequently, A SEQ500 SE50 kit set (purchased from MGI, 1000012551) was used to load the DNBs onto an SEQ500 sequencing chip (purchased from MGI).

In the sequencing chip, the MDA reagent in A PE50 sequencing kit (purchased from MGI, 1000012554) was added, incubated at 37°C for 30 minutes, and then the chip was washed with 5X SSC.

(3) The surface of the chip was modified with N3-PEG3500-NHS (the modification reagent was purchased from sigma, Cat. No.: JKA5086). After incubation for 30 minutes, DBCO-modified primer for chip sequence synthesis (the sequence was shown in SEQ ID NO: 3) was pumped therein, and incubated overnight at room temperature.

3. Sequencing and decoding of positional sequence information: Sequencing was performed according to the instructions of the BGISEQ500 SE50 sequencing kit, and the length of SE read was set as 25 bp. The fq file generated by the sequencing was stored for later use.

4. Extension of consensus sequence X2 (SEQ ID NO: 5) of chip sequence: Based on the above step 3, the cPAS reaction of 13 bases was continued to obtain a chip sequence (SEQ ID NO:8, which comprised a consensus sequence X1 (SEQ ID NO: 4), a tag sequence Y, and a consensus sequence X2 (SEQ ID NO: 5)).

5. Chip cutting: The prepared chip was cut into several small pieces. The size of the pieces was adjusted according to the experimental needs. The chip was immersed in 50mM tris buffer of pH8.0 at 4°C for later use.

### Example 2: In situ synthesis of cDNA

### 1. Synthesis of cDNA

Mouse tissue sections were prepared according to the standard method of frozen sections, and the frozen sections were attached to the chip prepared in Example 1. After being fixed with frozen methanol for 30 minutes, the tissues were permeabilized using 0.5% triton x-100. 5X SSC was used to wash the chip twice at room temperature, 200 µL of the reverse transcriptase reaction system as shown in Table 3 was prepared, the reaction solution was added to the chip to fully cover it, and the reaction was carried out at 42°C for 90-180 mins. The synthesis of cDNA was carried out by a reverse transcriptase using mRNA as a template and a polyT-containing primer (the sequence was shown in SEQ ID NO: 6, which comprised the consensus sequence A (CA), UMI sequence and polyT sequence), and a CCC overhang was added at 3'-end of the cDNA strand. After hybridization and annealing of TSO sequence (SEQ ID NO:7, which comprised the consensus sequence B (CB) and the GGG overhang) to the cDNA strand (through complementary pairing of the GGG at the end of the TSO sequence and the CCC overhang of the cDNA strand), the cDNA strand was continuously extended by reverse transcriptase using the consensus sequence B as a template, so that the 3'-end of the cDNA was labeled with a c(CB) tag (a complementary sequence of the consensus sequence B).

**Table 3: Synthesis system of cDNA**

| Ingredient | Volume (µL) | Final concentration |
|---|---|---|
| Superscript II First strand buffer (5X) (Purchased from thermo fisher, Cat. No.: 18064022) | 40 | 1X |
| Betaine (5 M) (Purchased from Aladdin, Cat. No.: B 105554) | 40 | 1M |
| dNTP (10 mM) | 20 | 1 mM |
| MgCl₂ (100 mM) | 15 | 7.5 mM |
| TSO sequence (SEQ ID NO:7) (synthesized by Liuhe BGI) | 10 | 1 µM |
| polyT primer (SEQ ID NO:6) (comprising 5'-end phosphorylation modification) (Synthesized by Liuhe BGI) | 10 | 1 µM |
| Superscript II RT (200U/µL) (purchased from thermo fisher, Cat. No.: 18064022) | 10 | 10U/µL |
| DTT (100 mM) | 10 | 5 mM |
| RNase inhibitor (40U/µL) (purchased from thermo fisher, Cat. No.: N8080119) | 5 | 1U/µL |
| NF H₂O | 40 | - |

The synthesized cDNA strand comprised the following sequence: reverse transcription primer sequence (SEQ ID NO: 6) - cDNA sequence - c(TSO) sequence (complementary sequence of SEQ ID NO: 7).

### 2. Ligation of chip sequence of sequencing chip to cDNA

After cDNA synthesis, the chip was washed twice with 5X SSC, 1 mL of the reaction system as shown in Table 4 was prepared, an appropriate volume thereof was pumped into the chip to ensure that the chip was filled with the following ligation reaction solution, and the reaction was carried out at room temperature for 30 minutes.

Through the above reaction, the 5'-end of the cDNA sequence was ligated to the 3'-end of single-cell sequencing chip sequence (i.e., the 5'-end of the cDNA sequence was labeled with the chip sequence) to obtain a new nucleic acid molecule comprising positional information (i.e., tag sequence Y), which comprised the following sequence structure: chip sequence (SEQ ID NO: 8) - reverse transcription primer sequence (SEQ ID NO: 6) - cDNA sequence - c(TSO) sequence (complementary sequence of SEQ ID NO: 7).

After the reaction was completed, the chip was washed with 5X SSC. 200 µL of Bst polymerization reaction solution (NEB, M0275S) was prepared according to the instructions, pumped into the chip, and reacted at 65°C for 60 minutes to obtain position information-containing single-stranded nucleic acid molecules.

**Table 4: Ligation system**

| Ingredient | Volume (µL) | Final concentration |
|---|---|---|
| 10X T4 ligase buffer (purchased from NEB, Cat. No.: M0202S) | 100 | 1X |
| T4 ligase (purchased from NEB, Cat. No.: M0202S) | 100 | 60U/µL |
| Bridging oligonucleotide (100 µM, comprising 5'-end phosphorylation modification) (SEQ ID NO:9) | 10 | 1 µM |
| (synthesized by Liuhe BGI) | | |
| Glycerin | 10 | 10% |
| H₂O | 780 | - |

### 3. Release of cDNA

75µL of 80mM KOH was used to incubate the chip at room temperature for 5 minutes. After the liquid was collected, 10µL of 1M pH8.0 Tris-HCl was added to neutralize the cDNA recovery solution.

### 4. Amplification of cDNA

200 µL of the reaction system as shown in Table 5 was prepared, used for 3' transcriptome sequencing and library construction respectively, and divided into 2 tubes for PCR:

**Table 5: Amplification system of cDNA**

| Ingredient | Volume (µL) | Final concentration |
|---|---|---|
| cDNA recovery product | 84 | - |
| Primer 1 (SEQ ID NO: 10) (synthesized by Liuhe BGI) | 8 | 0.8 µM |
| Primer 2 (SEQ ID NO: 11) (synthesized by Liuhe BGI) | 8 | 0.8 µM |
| 2x HiFi (NEB, E2621S) | 100 | 1X |

The above reaction system was placed into a PCR machine and the reaction program was set as follows: 95°C 3min, 11 cycles (98°C 20s, 58°C 20s, 72°C 3min), 72°C 5min, 4°C ∞. After the reaction was completed, XP beads (purchased from AMPure) were used for magnetic bead-based purification and recovery. The dsDNA concentration was quantified using a Qubit instrument, and the length distribution of the cDNA amplification product was detected using a 2100 Bioanalyzer (purchased from Agilent). The detection results were shown in Fig. 6.

### Example 3: Library construction and sequencing of cDNA

### 1. Tn5 fragmentation

According to the cDNA concentration, 20ng of cDNA (obtained in step 4 of Example 2) was taken, added with 0.5 µM of Tn5 transposase and corresponding buffer (purchased from BGI, Cat. No.: 10000028493; the coating of Tn5 transposase was operated according to the instruction of Stereomics library preparation kit-S1), mixed well to obtain a 20 µL reaction system, the reaction was carried out at 55°C for 10 minutes, then 5 µL of 0.1% SDS was added and mixed well at room temperature for 5 minutes to terminate the Tn5 transposition.

### 2. PCR amplification

100µL of the following reaction system was prepared:

**Table 6: Reaction system for library construction and amplification**

| Ingredient | Volume (µL) | Final concentration |
|---|---|---|
| Product after Tn5 transposition | 25 | - |
| 2X Hifi ready mix | 50 | 0.8 µM |
| Library construction primer 1 (10 µM, comprising phosphorylation modification at 5'-end and phosphorothioate modification of the last two nucleotides at 3'-end) (SEQ ID NO: 12) | 4 | 0.4 µM |
| (synthesized by Liuhe BGI) | | |
| Library construction primer 2 (10 µM) (SEQ ID NO: 13) | 4 | 0.4 µM |
| (synthesized by Liuhe BGI) | | |
| NF H₂O | 17 | - |

After mixing, it was placed in a PCR machine and the following program was set as follows: 95°C 3min, 11 cycles (98°C 20s, 58°C 20s, 72°C 3min), 72°C 5min, 4°C ∞. After the reaction was completed, XP beads were used for magnetic bead-based purification and recovery. The dsDNA concentration was quantified using a Qubit instrument.

### 3. Sequencing

80 fmol of the above-fragmented amplification product was taken to prepare DNBs. 40µL of reaction system was prepared as follows:

**Table 7: DNB preparation system for sequencing**

| Ingredient | Volume (µL) | Final concentration |
|---|---|---|
| Amplification product of step 2 above | X (80 fmol) | - |
| 10X phi29 buffer (Obtained from Thermofisher, Cat. No.: B62) | 4 | 1X |
| 10 µM primer sequence (SEQ ID NO: 14) used to prepare DNBs for sequencing | 4 | 1 µM |
| H₂O | 32-x | - |

The above reaction volume was placed in a PCR machine for reaction, and the reaction conditions were as follows: 95°C for 3 minutes, 40°C for 3 minutes. After the reaction was completed, the resulting reaction solution was placed on ice and added with 40 µL of the mixed enzyme I required for DNB preparation in the DNBSEQ sequencing kit, 2 µL of the mix enzyme II, 1 µL of ATP, and 0.1 µL of T4 ligase. After mixing, the above reaction system was placed in a PCR machine and reacted at 30°C for 20 minutes to form DNBs.

According to the method described in the PE50 kit supporting MGISEQ2000, the DNBs were loaded onto the sequencing chip of MGISEQ2000, and sequencing was performed according to the relevant instructions. PE50 sequencing model was selected, in which the first-strand sequencing was divided into two sections of sequencing, and 25 bp was measured first and then underwent 15 cycles of dark reaction, then 10bp UMI sequence was measured, and the second-strand sequencing was set to measure 50bp.

### Example 4: Analysis of data

By alignment, the first-strand 25bp sequence obtained by cDNA sequencing was matched with the fq of the positioning sequence on the chip (the sequencing result in Example 1), and the 25bp matching reads in the two sequencing were extracted for subsequent analysis. For the 25bp matching reads in the two sequencing, its second-strand was analyzed, and the sequence of the second-strand was aligned to the mouse genome to extract the unique mapping reads.

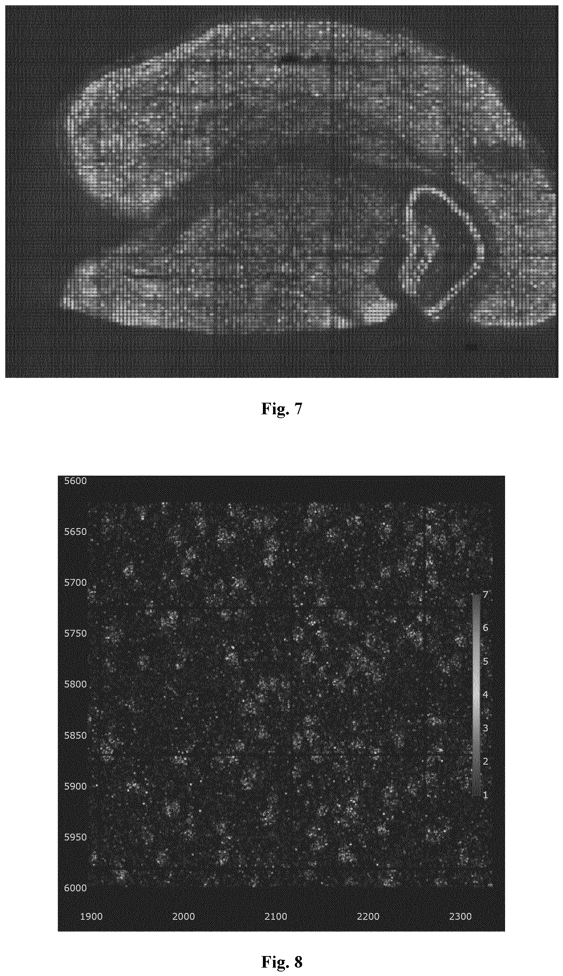

The unique mapping reads were mapped to the spatial positions of the chip to obtain a mouse brain gene expression map as shown in Fig. 7; when Fig. 7 was zoomed to a resolution of DNB level (i.e. nanometer level) (Fig. 8), it could been seen that there were obvious aggregations of single cells in the tissue sample, which indicated that the method of the present application could reduce the diffusion of RNA, allowing the detection of spatial transcriptome to reach nanometer-level resolution.

Statistics on the average numbers of genes and UMIs captured per 40,000 DNBs showed that an average of nearly 4,000 genes and 10,000 mRNA molecules could be captured, indicating that the method of the present application could capture sufficient numbers of genes and mRNA molecules for downstream analysis.

Although the specific embodiments of the present application have been described in detail, those skilled in the art will understand that various modifications and changes can be made to the details based on all teachings that have been disclosed, and these changes are all within the protection scope of the present application. The full scope of the present application is given by the appended claims and any equivalents thereof.

## Claims

1. A method for generating a labeled nucleic acid molecule population, which comprises the following steps:
(1) providing a biological sample and a nucleic acid array, wherein the nucleic acid array comprises a solid support, the solid support is coupled with multiple kinds of oligonucleotide probes, each kind of oligonucleotide probe comprises at least one copy; and the oligonucleotide probe in the direction from 5' to 3' comprises or consists of: a consensus sequence X1, a tag sequence Y, and a consensus sequence X2, wherein,
each kind of oligonucleotide probe has a different tag sequence Y, and the tag sequence Y has a nucleotide sequence unique to the position of the kind of oligonucleotide probe on the solid support;
(2) contacting the biological sample with the nucleic acid array so that the position of an RNA (e.g., mRNA) in the biological sample is mapped to the position of the oligonucleotide probe on the nucleic acid array; preprocessing the RNA (e.g., mRNA) in the biological sample to generate a first nucleic acid molecule population, wherein the preprocessing comprises:
(i) using a primer A to perform reverse transcription of the RNA (e.g., mRNA) of the biological sample to generate an extension product as a first nucleic acid molecule to be labeled, thereby generating the first nucleic acid molecule population; wherein, the primer A comprises a consensus sequence A and a capture sequence A, the capture sequence A is capable of annealing to the RNA (e.g., mRNA) to be captured and initiating an extension reaction, and the consensus sequence A is located upstream of the capture sequence A (e.g., located at the 5'-end of the primer A);
or,
(ii) (a) using a primer A to perform reverse transcription of the RNA (e.g., mRNA) of the biological sample to generate a cDNA strand, the cDNA strand comprising a cDNA sequence, which is generated by reverse transcription primed by the primer A and is complementary to the RNA (e.g., mRNA), and a 3'-end overhang; wherein, the primer A comprises a consensus sequence A and a capture sequence A, and the capture sequence A is capable of annealing to the RNA (e.g., mRNA) to be captured and initiating an extension reaction; the consensus sequence A is located upstream of the capture sequence A (e.g., at the 5'-end of the primer A); and, (b) annealing a primer B to the cDNA strand generated in (a) and performing an extension reaction to generate a first extension product as a first nucleic acid molecule to be labeled, thereby generating the first nucleic acid molecule population; wherein, the primer B comprises a consensus sequence B, a complementary sequence of the 3'-end overhang, and optionally a tag sequence B; the complementary sequence of the 3'-end overhang is located at the 3'-end of the primer B; and the consensus sequence B is located upstream of the complementary sequence of the 3'-end overhang (e.g., located at the 5'-end of the primer B);
or,
(iii) (a) using a primer A' to perform reverse transcription of the RNA (e.g., mRNA) of the biological sample to generate a cDNA strand, the cDNA strand comprises a cDNA sequence, which is generated by reverse transcription primed by the primer A' and is complementary to the RNA (e.g., mRNA), and a 3'-end overhang; wherein, the primer A' comprises a capture sequence A, the capture sequence A is capable of annealing to the RNA (e.g., mRNA) to be captured and initiating an extension reaction; (b) annealing a primer B to the cDNA strand generated in (a), and performing an extension reaction to generate a first extension product; wherein, the primer B comprises a consensus sequence B, a complementary sequence of the 3'-end overhang, and optionally a tag sequence B; the complementary sequence of the 3'-end overhang is located at the 3'-end of the primer B; the consensus sequence B is located upstream of the complementary sequence of the 3'-end overhang (e.g., located at the 5'-end of the primer B); and, (c) providing an extension primer and using the first extension product as a template to perform an extension reaction to generate a second extension product as a first nucleic acid molecule to be labeled, thereby generating the first nucleic acid molecule population;
(3) contacting a bridging oligonucleotide with the product of step (2) under a condition allowing annealing, annealing (e.g., in-situ annealing) the bridging oligonucleotide to the oligonucleotide probe and the first nucleic acid molecule to be labeled which is at the corresponding position of the oligonucleotide probe, and ligating the first nucleic acid molecule and the oligonucleotide probe on the array annealed with the bridging oligonucleotide to obtain a ligation product as a second nucleic acid molecule with a positioning tag, thereby generating a second nucleic acid molecule population;
wherein, the bridging oligonucleotide comprises: a first region and a second region, and optionally a third region located between the first region and the second region, the first region is located upstream of the second region (e.g., located 5' of the second region); wherein,
the first region is capable of annealing to the whole or a part of the consensus sequence A of the primer A in step (2)(i) or step (2)(ii) or to the whole or a part of the consensus sequence B of the primer B in step (2)(iii);
the second region is capable of annealing to the whole or a part of the consensus sequence X2.

2. The method according to claim 1, wherein, in step (3), when the first region and the second region of the bridging oligonucleotide are directly adjacent, the ligation of the first nucleic acid molecule to the oligonucleotide probe comprises: using a nucleic acid ligase to ligate the nucleic acid molecule hybridized with the first region and the nucleic acid molecule hybridized with the second region on the same bridging oligonucleotide, to obtain a ligation product as the second nucleic acid molecule with a positioning tag; or,
when the bridging oligonucleotide comprises the first region, the second region and the third region located between them, the ligation of the first nucleic acid molecule to the oligonucleotide probe comprises: using a nucleic acid polymerase to perform a polymerization reaction with the third region as a template, and using a nucleic acid ligase to ligate the nucleic acid molecule hybridized with the first region and the nucleic acid molecule hybridized with the third region and the second region on the same bridging oligonucleotide, to obtain a ligation product as the second nucleic acid molecule with a positioning tag; preferably, the nucleic acid polymerase does not have 5' to 3' exonucleolytic activity or strand displacement activity.

3. The method according to claim 1 or 2, which comprises step (1), step (2) (i) and step (3); wherein the ligation product obtained in step (3) is taken as the second nucleic acid molecule with a positioning tag, which comprises from 5' to 3': the consensus sequence X1, the tag sequence Y, the consensus sequence X2, optionally a complementary sequence of the third region of the bridging oligonucleotide, and the sequence of the first nucleic acid molecule to be labeled.

4. The method according to claim 3, wherein in step (2)(i), the capture sequence A is a random oligonucleotide sequence;
preferably, in step (3), the ligation product derived from each copy of the same kind of oligonucleotide probe has a different capture sequence A, and the capture sequence A serves as a unique molecular identifier (UMI) of the second nucleic acid molecule;
preferably, the extension product (the first nucleic acid molecule to be labeled) in step (2)(i) comprises from 5' to 3': the consensus sequence A, a cDNA sequence that is generated by reverse transcription primed by the primer A and is complementary to the RNA.

5. The method according to claim 3, wherein, in step (2)(i), the capture sequence A is a poly(T) sequence or a specific sequence for a target nucleic acid;
preferably, the primer A further comprises a tag sequence A, such as a random oligonucleotide sequence, and the tag sequence A serves as a unique molecular identifier (UMI) of the second nucleic acid molecule;
preferably, the capture sequence A is located at the 3'-end of the primer A, and the consensus sequence A is located upstream of the tag sequence A (e.g., located at the 5'-end of the primer A);
preferably, in step (3), the ligation product derived from each copy of the same kind of oligonucleotide probe has a different tag sequence A as a UMI;
preferably, the extension product in step (2)(i) comprises from 5' to 3': the consensus sequence A, the tag sequence A, and a cDNA sequence that is generated by reverse transcription primed by the primer A and is complementary to the RNA.

6. The method according to claim 1 or 2, which comprises step (1), step (2) (ii) and step (3); wherein the ligation product obtained in step (3) is taken as the second nucleic acid molecule with a positioning tag, which comprises from 5' to 3': the consensus sequence X1, the tag sequence Y, the consensus sequence X2, optionally a complementary sequence of the third region of the bridging oligonucleotide, and the sequence of the first nucleic acid molecule to be labeled.

7. The method according to claim 6, wherein, in step (2)(ii)(a), the capture sequence A is a random oligonucleotide sequence;
preferably, in step (3), the ligation product derived from each copy of the same kind of oligonucleotide probe has a different capture sequence A, and the capture sequence A serves as a unique molecular identifier (UMI) of the second nucleic acid molecule;
preferably, the first extension product (the first nucleic acid molecule to be labeled) in step (2)(ii) comprises from 5' to 3': the consensus sequence A, a cDNA sequence that is generated by reverse transcription primed by the primer A and is complementary to the RNA, the 3'-end overhang sequence, optionally a complementary sequence of the tag sequence B, and a complementary sequence of the consensus sequence B.

8. The method according to claim 6, wherein, in step (2)(ii)(a), the capture sequence A is a poly(T) sequence or a specific sequence for a target nucleic acid;
preferably, the primer A further comprises a tag sequence A, such as a random oligonucleotide sequence, and the tag sequence A serves as a unique molecular identifier (UMI) of the second nucleic acid molecule;
preferably, the capture sequence A is located at the 3'-end of the primer A, and the consensus sequence A is located upstream of the tag sequence A (e.g., located at the 5'-end of the primer A);
preferably, in step (3), the ligation product derived from each copy of the same kind of oligonucleotide probe has a different tag sequence A as a UMI;
preferably, the first extension product (the first nucleic acid molecule to be labeled) in step (2)(ii) comprises from 5' to 3': the consensus sequence A, the tag sequence A, a cDNA sequence that is generated by reverse transcription primed by the primer A and is complementary to the RNA, the 3'-end overhang sequence, optionally a complementary sequence of the tag sequence B, and a complementary sequence of the consensus sequence B.

9. The method according to any one of claims 3 to 8, wherein the primer A comprises a 5' phosphate at the 5'-end.

10. The method according to any one of claims 3 to 9, wherein before step (3), the method further comprises: processing the product of step (2)(i) or step (2)(ii) to remove RNA.

11. The method according to claim 1 or 2, which comprises step (1), step (2)(iii) and step (3); wherein, the ligation product obtained in step (3) is taken as the second nucleic acid molecule with a positioning tag, which comprises from 5' to 3': the consensus sequence X1, the tag sequence Y, the consensus sequence X2, optionally a complementary sequence of the third region of the bridging oligonucleotide, and the sequence of the first nucleic acid molecule sequence to be labeled.

12. The method according to claim 11, wherein in step (2)(iii)(c), the extension primer is the primer B or a primer B', and the primer B' is capable of annealing to the whole or a part of a complementary sequence of the consensus sequence B and initiating an extension reaction;
preferably, in step (2)(iii)(c), the extension primer is the primer B'.

13. The method according to claim 11 or 12, wherein, in step (2)(iii)(a), the capture sequence A of the primer A' is a random oligonucleotide sequence;
preferably, in step (2)(iii)(b), the primer B comprises the consensus sequence B, a complementary sequence of the 3'-end overhang, and the tag sequence B;
preferably, the first extension product comprises from 5' to 3': a cDNA sequence that is generated by reverse transcription primed by the primer A' and is complementary to the RNA sequence, the 3'-end overhang sequence, a complementary sequence of the tag sequence B, a complementary sequence of the consensus sequence B; wherein the complementary sequence of the tag sequence B serves as a unique molecular identifier (UMI) of the second nucleic acid molecule;
preferably, in step (2)(iii)(c), the second extension product (the first nucleic acid molecule to be labeled) comprises from 5' to 3': the consensus sequence B or its 3'-end partial sequence, the tag sequence B, a complementary sequence of the 3'-end overhang sequence, a complementary sequence of the cDNA sequence in the first extension product; wherein the tag sequence B serves as a unique molecular identifier (UMI) of the second nucleic acid molecule.

14. The method according to claim 11 or 12, wherein, in step (2)(iii)(a), the capture sequence A of the primer A' is a poly(T) sequence or a specific sequence for a target nucleic acid;
preferably, the primer A' further comprises a tag sequence A, such as a random oligonucleotide sequence, and a consensus sequence A;
preferably, the capture sequence A is located at the 3'-end of the primer A';
preferably, the consensus sequence A is located upstream of the capture sequence A (e.g., located at the 5'-end of the primer A');
preferably, the primer B comprises a consensus sequence B, a complementary sequence of the 3'-end overhang, and a tag sequence B;
preferably, in step (2)(iii)(b), the first extension product comprises from 5' to 3': the consensus sequence A, optionally the tag sequence A, a cDNA sequence that is generated by reverse transcription primed by the primer A' and is complementary to the RNA, the 3'-end overhang sequence, a complementary sequence of the tag sequence B, and a complementary sequence of the consensus sequence B;
preferably, in step (2)(iii)(c), the second extension product (the first nucleic acid molecule to be labeled) comprises from 5' to 3': the consensus sequence B or its 3'-end partial sequence, the tag sequence B, a complementary sequence of the 3'-end overhang sequence, a complementary sequence of the cDNA sequence in the first extension product, optionally a complementary sequence of the tag sequence A, and a complementary sequence of the consensus sequence A;
preferably, in step (3), the ligation product derived from each copy of the same kind of oligonucleotide probe has a different tag sequence B as a UMI.

15. The method according to any one of claims 11 to 14, wherein the extension primer comprises a 5' phosphate at the 5'-end.

16. The method according to any one of claims 11 to 15, wherein, before step (2)(iii)(c), the method further comprises: processing the product of step (2)(iii)(a) or step (2)(iii)(b) to remove RNA.

17. The method according to any one of claims 11 to 16, wherein in step (2)(iii)(b), the cDNA strand is annealed through its 3'-end overhang to the primer B, and, the cDNA strand is extended using the primer B as a template to generate the first extension product under the presence of a nucleic acid polymerase (e.g., a DNA polymerase or a reverse transcriptase).

18. The method according to any one of claims 11 to 17, wherein the 3'-end overhang has a length of at least 1, at least 2, at least 3, at least 4, at least 5, at least 6, at least 7, at least 8, at least 9, at least 10 or more nucleotides; preferably, the 3'-end overhang is a 3'-end overhang of 2-5 cytosine nucleotides (e.g., a CCC overhang).

19. The method according to any one of claims 1 to 18, wherein in step (2), the biological sample is permeabilized before the preprocessing.

20. The method according to any one of claims 1 to 19, wherein the biological sample is a tissue sample;
preferably, the tissue sample is a tissue section;
preferably, the tissue section is prepared from a fixed tissue, for example, a formalin-fixed paraffin-embedded (FFPE) tissue or a deep-frozen tissue.

21. The method according to any one of claims 1 to 20, wherein in step (2), the reverse transcription is performed by using a reverse transcriptase;
preferably, the reverse transcriptase has terminal deoxynucleotidyl transferase activity; preferably, the reverse transcriptase is capable of using an RNA (e.g., mRNA) as a template to synthesize a cDNA strand, and adding an overhang at the 3'-end of the cDNA strand;
preferably, the reverse transcriptase is capable of adding an overhang having a length of at least 1, at least 2, at least 3, at least 4, at least 5, at least 6, at least 7, at least 8, at least 9, at least 10 or more nucleotides at the 3'-end of the cDNA strand;
preferably, the reverse transcriptase is capable of adding an overhang of 2-5 cytosine nucleotides (e.g., a CCC overhang) at the 3'-end of the cDNA strand;
preferably, the reverse transcriptase is selected from the group consisting of M-MLV reverse transcriptase, HIV-1 reverse transcriptase, AMV reverse transcriptase, telomerase reverse transcriptase, and variants, modified products and derivatives thereof with the reverse transcription activity of the above-mentioned reverse transcriptases.

22. The method according to any one of claims 1 to 21, wherein steps (2) and (3) have one or more characteristics selected from the following:
(1) the primer A, primer A', primer B, and bridging oligonucleotide each independently comprise or consist of natural nucleotides (e.g., deoxyribonucleotides or ribonucleotides), modified nucleotides, non-natural nucleotides, or any combination thereof; preferably, the primer A and primer A' can initiate an extension reaction;
(2) the primer B comprises a modified nucleotide (e.g., locked nucleic acid); preferably, the primer B comprises one or more modified nucleotides (e.g., locked nucleic acid) at the 3'-end;
(3) the tag sequence A and tag sequence B each independently have a length of 5-200nt (e.g., 5-30nt, 6-15nt);
(4) the consensus sequence A and consensus sequence B each independently have a length of 10-200nt (e.g., 10-100nt, 20-100nt, 25-100nt, 5-10nt, 10-15nt, 15-20nt, 20-30nt, 30-40 nt, 40-50 nt, 50-100nt);
(5) the primer A, primer A', and primer B each independently have a length of 10-200 nt (e.g., 10-20 nt, 20-30 nt, 30-40 nt, 40-50 nt, 50-100 nt, 100-150 nt, 150-200 nt);
(6) the first region and second region of the bridging oligonucleotide each independently have a length of 3-100 nt (e.g., 3-10 nt, 10-15 nt, 15-20 nt, 20-30 nt, 30-40 nt, 40-50 nt, 50-100 nt);
(7) the third region of the bridging oligonucleotide has a length of 0-100nt (e.g., 0-10nt, 10-15nt, 15-20nt, 20-30nt, 30-40nt, 40-50nt, 50-100nt);
(8) the bridging oligonucleotide has a length of 6-200nt (e.g., 20-70nt, 6-15nt, 15-20nt, 20-30nt, 30-40nt, 40-50nt, 50-100nt, 100-150 nt, 150-200 nt);
(9) the poly(T) sequence comprises at least 10, or at least 20 (e.g., at least 30) deoxythymidine residues;
(10) the random oligonucleotide sequence has a length of 5-200nt (e.g., 5-30nt, 6-15nt).

23. The method according to any one of claims 1 to 22, wherein the method further comprises: (4) recovering and purifying the second nucleic acid molecule population.

24. The method according to any one of claims 1 to 23, wherein the obtained second nucleic acid molecule population and/or complement thereof is used to construct a transcriptome library or for transcriptome sequencing.

25. The method according to any one of claims 1 to 24, wherein the oligonucleotide probe in step (1) has one or more characteristics selected from the following:
(1) the consensus sequence X1, tag sequence Y, and consensus sequence X2 each independently comprises natural nucleotides (e.g., deoxyribonucleotides or ribonucleotides), modified nucleotides, non-natural nucleotides (e.g., peptide nucleic acid (PNA) or locked nucleic acid), or any combination thereof; preferably, the consensus sequence X2 has a free hydroxyl group (-OH) at the 3'-end;
(2) the consensus sequence X1, tag sequence Y and consensus sequence X2 each independently have a length of 2-100nt (e.g., 10-200nt, 10-100nt, 20-100nt, 25-100nt, 50-100nt, 5-30nt, 6-15nt, 5-10nt, 10-15nt, 15-20nt, 20-30nt, 30-40nt, 40-50nt);
(3) the oligonucleotide probes each independently have a length of 15-200nt (e.g., 15-20nt, 20-30nt, 30-40nt, 40-50nt, 50-100nt, 100-150nt, 150-200nt).

26. The method according to any one of claims 1 to 25, wherein the oligonucleotide probe is coupled to the solid support through a linker;
preferably, the linker is a linking group capable of coupling with an activating group, and the surface of the solid support is modified with the activating group;
preferably, the linker comprises -SH, -DBCO, or -NHS;
preferably, the linker is -DBCO, and the surface of the solid support is modified with 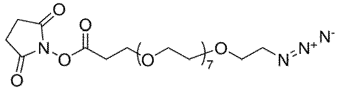 (Azido-dPEG^{®} 8-NHS ester).

27. The method according to any one of claims 1 to 26, wherein the nucleic acid array of step (1) has one or more characteristics selected from the following:
(1) the oligonucleotide probes coupled to the same solid support have the same consensus sequence X1 and/or the same consensus sequence X2;
(2) the consensus sequence X1 of the oligonucleotide probes comprises a cleavage site; preferably, the cleavage site can be cleaved or broken by a method selected from nicking enzyme digestion, USER enzyme digestion, light-responsive excision, chemical excision or CRISPR-mediated excision.

28. The method according to any one of claims 1 to 27, wherein the nucleic acid array of step (1) is provided by the steps comprising:
(1) providing multiple kinds of carrier sequences, each kind of carrier sequence comprises at least one copy of the carrier sequence, and the carrier sequence comprises in the direction from 5' to 3': a complementary sequence of the consensus sequence X2, a complementary sequence of the tag sequence Y, and an immobilization sequence; wherein, the complementary sequence of the tag sequence Y of each kind of carrier sequence is different from each other;
(2) attaching the multiple kinds of carrier sequences to the surface of a solid support (e.g., a chip);
(3) providing an immobilization primer, and using the carrier sequence as a template to perform a primer extension reaction to generate an extension product, so as to obtain the oligonucleotide probe; wherein the immobilization primer comprises the sequence of the consensus sequence X1, and is capable of annealing to the immobilization sequence of the carrier sequence and initiating an extension reaction; preferably, the extension product in the direction from 5' to 3' comprises or consists of: the consensus sequence X1, the tag sequence Y, and the consensus sequence X2;
(4) linking the immobilization primer to the surface of the solid support; wherein step (3) and step (4) are performed in any order;
(5) optionally, the immobilization sequence of the carrier sequence further comprises a cleavage site, and the cleavage can be selected from the group consisting of nicking enzyme digestion, USER enzyme digestion, light-responsive excision, chemical excision and CRISPR-mediated excision; performing cleavage at the cleavage site comprised in the immobilization sequence of the carrier sequence to digest the carrier sequence, so that the extension product in step (3) is separated from the template (i.e., the carrier sequence) from which the extension product is generated, thereby linking the oligonucleotide probe to the surface of the solid support (e.g., chip);
preferably, each kind of carrier sequence is a DNB formed by a concatemer of multiple copies of the carrier sequence;
preferably, the multiple kinds of carrier sequences are provided in step (1) through the following steps:
(i) providing multiple kinds of carrier-template sequences, the carrier-template sequence comprises a complementary sequence of a carrier sequence;
(ii) using each kind of the carrier-template sequence as a template to perform a nucleic acid amplification reaction to obtain an amplification product of each kind of carrier-template sequence, wherein the amplification product comprises at least one copy of the carrier sequence;
preferably, performing rolling circle replication to obtain a DNB formed by the concatemer of the carrier sequence.

29. The method according to any one of claims 1 to 28, wherein the solid support in step (1) has one or more characteristics selected from the following:
(1) the solid support is selected from the group consisting of latex bead, dextran bead, polystyrene surface, polypropylene surface, polyacrylamide gel, gold surface, glass surface, chip, sensor, electrode and silicon wafer; preferably, the solid support is a chip;
(2) the solid support is planar, spherical or porous;
(3) the solid support can be used as a sequencing platform, such as a sequencing chip; preferably, the solid support is a sequencing chip for Illumina, MGI or Thermo Fisher sequencing platform; and
(4) the solid support is capable of releasing the oligonucleotide probe spontaneously or upon exposure to one or more stimuli (e.g., temperature change, pH change, exposure to specific chemicals or phases, exposure to light, exposure to reducing agents, etc.).

30. A method of constructing a nucleic acid molecule library, comprising:
(a) generating a labeled nucleic acid molecule population by the method according to any one of claims 1 to 29;
(b) randomly fragmenting the nucleic acid molecules in the labeled nucleic acid molecule population and adding an adapter; and
(c) optionally, amplifying and/or enriching the product of step (b);
thereby obtaining the nucleic acid molecule library;
preferably, the nucleic acid molecule library is used for sequencing, such as transcriptome sequencing, such as single-cell transcriptome sequencing.

31. The method according to claim 30, wherein, before performing step (b), the method further comprises step (pre-b): amplifying and/or enriching the labeled nucleic acid molecule population;
preferably, in step (pre-b), the labeled nucleic acid molecule population is subjected to a nucleic acid amplification reaction to generate an amplification product.
preferably, the nucleic acid amplification reaction is performed using at least a primer C and/or a primer D, wherein the primer C is capable of hybridizing with or annealing to a complementary sequence of the consensus sequence X1 or a complementary sequence of the 3'-end sequence of the consensus sequence X1, and initiating an extension reaction; the primer D is capable of hybridizing with or annealing to the nucleic acid molecules in the labeled nucleic acid molecule population, and initiating an extension reaction;
preferably, the nucleic acid amplification reaction in step (pre-b) is performed using a nucleic acid polymerase (e.g., a DNA polymerase; for example, a DNA polymerase with strand displacement activity and/or high fidelity).

32. The method according to claim 31, wherein, in step (b), the nucleic acid molecules obtained in the previous step are randomly fragmented into fragments and adapters are added to both ends of the fragments, using a transposase;
preferably, the transposase is selected from the group consisting of Tn5 transposase, MuA transposase, Sleeping Beauty transposase, Mariner transposase, Tn7 transposase, Tn10 transposase, Ty1 transposase, and Tn552 transposase, as well as variants, modified products and derivatives thereof having the transposition activity of the above-mentioned transposases;
preferably, the transposase is Tn5 transposase;
preferably, in step (c), at least a primer C' and/or a primer D' are used to amplify the product of step (b); wherein the adapters at both ends of the fragment are a first adapter and a second adapter respectively, and the primer C' is capable of hybridizing with or annealing to the first adapter and initiating an extension reaction, and the primer D' is capable of hybridizing with or annealing to the second adapter and initiating an extension reaction.

33. A method for sequencing a nucleic acid sample, comprising:
(1) constructing a nucleic acid molecule library by the method according to any one of claims 30 to 32; and
(2) sequencing the nucleic acid molecule library.

34. A kit, which comprises:
(i) a nucleic acid array for labeling nucleic acids, which comprises a solid support, in which the solid support is coupled with multiple kinds of oligonucleotide probes; each kind of oligonucleotide probe comprises at least one copy; and, the oligonucleotide probe in the direction from 5' to 3' comprises or consists of: a consensus sequence X1, a tag sequence Y and a consensus sequence X2, wherein,
each kind of oligonucleotide probe has a different tag sequence Y, and the tag sequence Y has a nucleotide sequence unique to the position of the kind of oligonucleotide probe on the solid support;
(ii) a primer A, or a primer set comprising a primer A' and a primer B, or a primer set comprising a primer A and a primer B, wherein:
the primer A comprises a consensus sequence A and a capture sequence A, in which the capture sequence A is capable of annealing to an RNA (e.g., mRNA) to be captured and initiating an extension reaction; preferably, the consensus sequence A is located upstream of the capture sequence A (e.g., located at the 5'-end of the primer A);
the primer A' comprises a capture sequence A, in which the capture sequence A is capable of annealing to an RNA (e.g., mRNA) to be captured and initiating an extension reaction;
the primer B comprises a consensus sequence B, a complementary sequence of a 3'-end overhang, and optionally a tag sequence B; preferably, the complementary sequence of a 3'-end overhang is located at the 3'-end of the primer B; preferably, the consensus sequence B is located upstream of the complementary sequence of a 3'-end overhang (e.g., located at the 5'-end of the primer B); wherein the 3'-end overhang refers to one or more non-templated nucleotides comprised in the 3'-end of a cDNA strand generated by reverse transcription using the RNA captured by the capture sequence A of the primer A' as a template;
and,
(iii) a bridging oligonucleotide, which comprises: a first region and a second region, and optionally a third region located between the first region and the second region, in which the first region is located upstream of the second region (e.g., located 5' of the second region); wherein,
the first region is capable of: (a) annealing to the whole or a part of the consensus sequence A of the primer A; or (b) annealing to the whole or a part of the consensus sequence B of the primer B;
the second region is capable of annealing to the whole or a part of the consensus sequence X2.

35. The kit according to claim 34, comprising: a nucleic acid array for labeling nucleic acids as described in (i), a primer A as described in (ii), and a bridging oligonucleotide as described in (iii); wherein, the first region of the bridging oligonucleotide is capable of annealing to the whole or a part of the consensus sequence A of the primer A, and the second region of the bridging oligonucleotide is capable of annealing to the whole or a part of the consensus sequence X2;
preferably, the capture sequence A of the primer A is a random oligonucleotide sequence;
preferably, the capture sequence A of the primer A is a poly(T) sequence or a specific sequence for a target nucleic acid; preferably, the primer A further comprises a tag sequence A, such as a random oligonucleotide sequence; preferably, the capture sequence A is located at the 3'-end of the primer A, and the consensus sequence A is located upstream of the tag sequence A (e.g., located at the 5'-end of the primer A);
preferably, the primer A comprises a 5' phosphate at the 5'-end.

36. The kit according to claim 34, comprising: a nucleic acid array for labeling nucleic acids as described in (i), a primer set comprising a primer A' and a primer B as described in (ii), and, a bridging oligonucleotide as described in (iii); wherein the first region of the bridging oligonucleotide is capable of annealing to the whole or a part to the consensus sequence B of the primer B, and the second region of the bridging oligonucleotide is capable of annealing to the whole or a part of the consensus sequence X2;
preferably, the capture sequence A of the primer A' is a random oligonucleotide sequence;
preferably, the capture sequence A of the primer A' is a poly(T) sequence or a specific sequence for a target nucleic acid; preferably, the primer A' further comprises a tag sequence A, and a consensus sequence A; preferably, the capture sequence A is located at the 3'-end of the primer A'; preferably, the consensus sequence A is located upstream of the capture sequence A (e.g., located at the 5'-end of the primer A');
preferably, the primer B comprises a consensus sequence B, a complementary sequence of the 3'-end overhang, and a tag sequence B;
preferably, the kit further comprises a primer B', which is capable of annealing to the whole or a part of a complementary sequence of the consensus sequence B, and initiating an extension reaction;
preferably, the primer B or primer B' comprises a 5' phosphate at the 5'-end;
preferably, the primer B comprises a modified nucleotide (e.g., locked nucleic acid); preferably, the primer B comprises one or more modified nucleotides (e.g., locked nucleic acid) at the 3'-end.

37. The kit according to claim 34, which comprises: a nucleic acid array for labeling nucleic acids as described in (i), a primer set comprising a primer A and a primer B as described in (ii), and, a bridging oligonucleotide as described in (iii); wherein the first region of the bridging oligonucleotide is capable of annealing to the whole or a part of the consensus sequence A of the primer A, and the second region of the bridging oligonucleotide is capable of annealing to the whole or a part of the consensus sequence X2;
preferably, the capture sequence A of the primer A is a random oligonucleotide sequence;
preferably, the capture sequence A of the primer A is a poly(T) sequence or a specific sequence for a target nucleic acid; preferably, the primer A further comprises a tag sequence A, such as a random oligonucleotide sequence; preferably, the capture sequence A is located at the 3'-end of the primer A, and the consensus sequence A is located upstream of the tag sequence A (e.g., located at the 5'-end of the primer A);
preferably, the primer A comprises a 5' phosphate at the 5'-end;
preferably, the primer B comprises a modified nucleotide (e.g., locked nucleic acid); preferably, the primer B comprises one or more modified nucleotides (e.g., locked nucleic acid) at the 3'-end.

38. The kit according to any one of claims 34 to 37, which has one or more characteristics selected from the following:
(1) the oligonucleotide probe, primer A, primer A', primer B, primer B', and bridging oligonucleotide each independently comprise or consist of natural nucleotides (e.g., deoxyribonucleotides or ribonucleotides), modified nucleotides, non-natural nucleotides, or any combination thereof; preferably, the primer A, primer A', and primer B' is capable of initiating an extension reaction; preferably, the consensus sequence X2 has a free hydroxyl group (-OH) at the 3'-end;
(2) the oligonucleotide probes each independently have a length of 15-300nt (e.g., 15-200nt, 15-20nt, 20-30nt, 30-40nt, 40-50nt, 50-100nt, 100-150nt, 150-200nt);
(3) the primer A, primer A', primer B, and primer B' each independently have a length of 10-200 nt (e.g., 10-20 nt, 20-30 nt, 30-40 nt, 40-50 nt, 50-100 nt nt, 100-150 nt, 150-200 nt);
(4) the bridging oligonucleotide has a length of 6-200nt (e.g., 20-70nt, 6-15nt, 15-20nt, 20-30nt, 30-40nt, 40-50nt, 50-100nt, 100-150 nt, 150-200 nt);
(5) the oligonucleotide probes coupled to the same solid support have the same consensus sequence X1 and/or the same consensus sequence X2;
(6) the consensus sequence X1 of the oligonucleotide probes comprises a cleavage site; preferably, the cleavage site can be cleaved or broken by a method selected from nicking enzyme digestion, USER enzyme digestion, light-responsive excision, chemical excision or CRISPR-mediated excision.

39. The kit according to any one of claims 34 to 38, which further comprises a reverse transcriptase, a nucleic acid ligase, a nucleic acid polymerase and/or a transposase;
preferably, the reverse transcriptase has terminal deoxynucleotidyl transferase activity; preferably, the reverse transcriptase is capable of synthesizing a cDNA strand using an RNA (e.g., mRNA) as a template, and adding a 3'-end overhang to the 3'-end of the cDNA strand; preferably, the reverse transcriptase is capable of adding to the 3'-end of the cDNA strand an overhang having a length of at least 1, at least 2, at least 3, at least 4, at least 5, at least 6, at least 7, at least 8, at least 9, at least 10 or more nucleotides; preferably, the reverse transcriptase is capable of adding to the 3'-end of the cDNA strand an overhang of 2-5 cytosine nucleotides (e.g., CCC overhang); preferably, the reverse transcriptase is selected from the group consisting of M-MLV reverse transcriptase, HIV-1 reverse transcriptase, AMV reverse transcriptase, telomerase reverse transcriptase, and variants, modified products and derivatives thereof having the reverse transcription activity of the above-mentioned reverse transcriptases;
preferably, the nucleic acid polymerase does not have 5' to 3' exonucleolytic activity or strand displacement activity;
preferably, the transposase is selected from the group consisting of Tn5 transposase, MuA transposase, Sleeping Beauty transposase, Mariner transposase, Tn7 transposase, Tn10 transposase, Ty1 transposase, Tn552 transposase, as well as variants, modified products and derivatives thereof having the transposition activity of the above-mentioned transposases.

40. The kit according to any one of claims 34 to 39, which further comprises: a reagent for nucleic acid hybridization, a reagent for nucleic acid extension, a reagent for nucleic acid amplification, and a reagent for recovering or purifying nucleic acid, a reagent for constructing transcriptome sequencing library, a reagent for sequencing (e.g., second- or third-generation sequencing), or any combination thereof.

41. Use of the method according to any one of claims 1 to 29 or the kit according to any one of claims 34 to 40 for constructing a nucleic acid molecule library or for performing transcriptome sequencing.
